(19) Europäisches Patentamt
European Patent Office
Office européen des brevets

(11) EP 0 842 153 B1

(12) **EUROPÄISCHE PATENTSCHRIFT**

(45) Veröffentlichungstag und Bekanntmachung des Hinweises auf die Patenterteilung:
**30.05.2001 Patentblatt 2001/22**

(21) Anmeldenummer: **96929198.8**

(22) Anmeldetag: **02.08.1996**

(51) Int Cl.$^7$: **C07D 221/26**, C07D 211/78, C07D 211/70, C07C 229/34, C07D 211/74

(86) Internationale Anmeldenummer:
**PCT/EP96/03401**

(87) Internationale Veröffentlichungsnummer:
**WO 97/06146 (20.02.1997 Gazette 1997/09)**

(54) **VERFAHREN ZUR HERSTELLUNG VON NORBENZOMORPHAN EINER ZWISCHENSTUFE BEI HERSTELLUNG VON PHARMAZEUTISCH WERTVOLLEN BENZOMORPHANDERIVATEN, INSBESONDERE VON (-)-(1R,5S,2"R)-3'-HYDROXY-2-(2-METHOXYPROPYL)-5,9,9-TRIMETHYL-6,7-BENZOMORPHAN**

METHOD OF PREPARING NORBENZOMORPHANE AS AN INTERMEDIATE IN THE PREPARATION OF PHARMACEUTICALLY USEFUL BENZOMORPHANE DERIVATIVES, IN PARTICULAR (-)-(1R,5S,2"R)-3'-HYDROXY-2-(2-METHOXYPROPYL)-5,9,9-TRIMETHYL-6,7-BENZOMORPHANE

PROCEDE DE PREPARATION DE NORBENZOMORPHANE D'UN INTERMEDE LORS DE LA PREPARATION DE DERIVES DE BENZOMORPHANE INTERESSANTS SUR LE PLAN PHARMACEUTIQUE, NOTAMMENT DE (-)-(1R,5S,2"R)-3'-HYDROXY-2-(2-METHOXYPROPYLE)-5,9,9-TRIMETHYLE-6,7-BENZOMORPHANE

(84) Benannte Vertragsstaaten:
**AT BE CH DE DK ES FI FR GB GR IE IT LI LU MC NL PT SE**
Benannte Erstreckungsstaaten:
**SI**

(30) Priorität: **03.08.1995 DE 19528472**

(43) Veröffentlichungstag der Anmeldung:
**20.05.1998 Patentblatt 1998/21**

(73) Patentinhaber:
- **Boehringer Ingelheim Pharma KG**
  **55218 Ingelheim am Rhein (DE)**
  Benannte Vertragsstaaten:
  **BE CH DE DK ES FI FR GR IT LI LU MC NL PT SE AT**
- **Boehringer Ingelheim International GmbH**
  **55218 Ingelheim am Rhein (DE)**
  Benannte Vertragsstaaten:
  **GB IE**

(72) Erfinder:
- **GRAUERT, Matthias**
  **D-55218 Ingelheim am Rhein (DE)**
- **MERZ, Herbert**
  **D-55218 Ingelheim am Rhein (DE)**
- **BALTES, Hanfried**
  **D-55597 Wöllstein (DE)**

(74) Vertreter: **Laudien, Dieter, Dr.**
**Boehringer Ingelheim GmbH,**
**Binger Strasse 173**
**55216 Ingelheim (DE)**

(56) Entgegenhaltungen:
**EP-A- 0 028 717** **EP-A- 0 521 422**
**DE-A- 2 027 077**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 842 153 B1

## Beschreibung

Die vorliegende Erfindung betrifft ein Verfahren zur Herstellung von Norbenzomorphan - der zentralen Zwischenstufe bei der Herstellung von pharmazeutisch wertvollen Benzomorphanderivaten der allgemeinen Formel 1, insbesondere von (-)-(1 R,5S,2"R)-3'-Hydroxy-2-(2-methoxypropyl)-5,9,9-trimethyl--6,7-benzomorphan bzw. [(-)-(2R, 6S,2'R)-3-(2-Methoxypropyl)-6,11,11 -trimethyl--1,2,3,4,5,6-hexahydro-2,6-methano-benzo[α]oxacin-9-ol] (BIII 277).

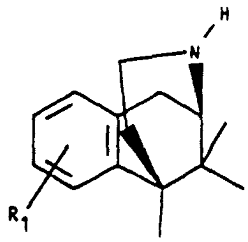

1

worin

$R_1$ Wasserstoff, $C_1$-$C_6$-Alkyl, Halogen. Hydroxy, $C_1$-$C_8$-Alkoxy, einen über ein Sauerstoff gebundenen Benzoylrest oder einen Alkylcarboxylrest mit einem geradkettigen oder verzweigten Niederalkylrest mit 1 bis 6 Kohlenstoffatomen - wobei der Alkylrest gegebenenfalls mit einem oder mehreren Halogenatom(en), die untereinander gleich oder verschieden sein können, substituiert sein kann -
Nitro, Cyano, $-NH_2$, -NH($C_1$-$C_8$-Alkyl), -N($C_1$-$C_8$-Alkyl$)_2$, wobei die Alkylreste gleich oder verschieden sein können, -NH-Acyl-($C_1$-$C_8$-Alkyl), worin Acyl für Benzoyl oder einen Alkylcarbonylrest mit einem geradkettigen oder verzweigten Niederalkylrest mit 1 bis 6 Kohlenstoffatom(en), wobei der Alkylrest gegebenenfalls mit einem oder mehreren Halogenatom(en), die untereinander gleich oder verschieden sein können, substituiert sein kann,

bedeuten kann.

Soweit nicht im einzelnen abweichende Angaben gemacht werden, werden die allgemeinen Definitionen im folgenden Sinn gebraucht:

$C_1$-$C_6$-Alkyl bzw. $C_1$-$C_8$-Alkyl steht im allgemeinen für einen verzweigten oder unverzweigten Kohlenwasserstoffrest mit 1 bis 6 bzw. 8 Kohlenstoffatom(en), der gegebenenfalls mit einem oder mehreren Halogenatom(en) - vorzugsweise Fluor - substituiert sein kann, die untereinander gleich oder verschieden sein können. Als Beispiele seien folgende Kohlenwasserstoffreste genannt:

Methyl, Ethyl, Propyl, 1-Methylethyl (Isopropyl), Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylproypyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl. Bevorzugt sind - sofern nicht anders angegeben - Niederalkylreste mit 1 bis 3 Kohlenstoffatomen, wie Methyl, Ethyl, Propyl, Isopropyl.

Acyl steht im allgemeinen für Benzoyl oder für Alkylcarbonylreste - wie geradkettiges oder verzweigtes Niederalkyl mit 1 bis 6 Kohlenwasserstoffatom(en) - die über eine Carbonylgruppe gebunden sind, wobei der Alkylrest gegebenenfalls mit einem oder mehreren Halogenatom(en), die untereinander gleich oder verschieden sein können, substituiert sein kann. Bevorzugt sind Alkylreste mit bis zu 4 Kohlenstoffatomen. Als Beispiele seien genannt: Acetyl, Trifluoracetyl, Ethylcarbonyl, Propylcarbonyl, Isopropylcarbonyl, Butylcarbonyl sowie Isobutylcarbonyl. Besonders bevorzugt ist der Acetylrest.

Die eingangs erwähnten Benzomorphanderivate verkörpern vielversprechende Wirkstoffe zur Behandlung neurodegenerativer Erkrankungen sowie Gehirnischaemien verschiedener Genese. Beispielsweise seien genannt: Status Epileptikus, Hypoglykämie, Hypoxie, Anoxie, Gehirntrauma, Gehirnoedem, amorphe laterale Sklerose, Huntington's Disease, Morbus Alzheimer, Hypotonie, Herzinfarkt, Gehirn-Schlaganfall und perinatale Asphyxie. Das Benzomorphanderivat mit der Chiffre BIII 277 sowie verwandte Benzomorpahne sind u.a. in der Deutschen Offenlegungsschrift DE-0S 41 21 821 eingehend beschrieben.

Daneben sind weitere Synthesestrategien zum Aufbau von Benzomorphanderivaten aus dem Stand der Technik bekannt [Deutsche Offenlegungsschrift 2 027 077, Europäische Offenlegungsschrift 0 004 960]. Allerdings werden - mit Ausnahme der DE-OS 41 21 821 - in diesen Publikationen lediglich Synthesen für die Racemate beschrieben,

bei deren Aufspaltung letztendlich 50% des nicht gewünschten Isomeren verworfen werden müssen. Daneben besteht u. a. bei einzelnen Reaktionsschritten die Gefahr der Bildung von Regioisomeren.

Es ist daher die Aufgabe der vorliegenden Erfindung, die Nachteile der aus dem Stand der Technik bekannten Verfahren zu überwinden und einen Herstellungsweg bereitzustellen, bei dem zum einen die Bildung von - gegebenenfalls auftretenden - Regioisomeren beim Aufbau des Benzomorphan-Grundgerüstes vermieden wird und das es zum anderen ermöglicht, das pharmakologisch wirksamere Stereoisomere in höheren Ausbeuten zu gewinnen.

Diese Aufgabe wird durch das im folgenden beschriebene Verfahren und insbesondere durch die in den Beispielen wiedergegebene Verfahrensschritte gelöst. Verschiedenartige, andere und weitere Merkmale, Ausgestaltungen des Verfahrens und dergleichen, die der vorliegenden Erfindung zugeordnet sind, werden für den Fachmann aus der vorliegenden Beschreibung ersichtlich und in Verbindung mit den Beispielen, in welchen die gegenwärtig bevorzugten Ausführungsformen der vorliegenden Erfindung beispielhaft dargestellt sind, noch besser verständlich. - Es wird jedoch ausdrücklich darauf hingewiesen , daß die Beispiele und die diesen zugeordnete Beschreibung lediglich zum Zwecke der Erläuterung und Beschreibung vorgesehen sind.

Im Gegensatz zu den aus dem Stand der Technik bekannten Verfahren wird durch die vorliegende Erfindung ein verbesserter Herstellungsweg vorgeschlagen, bei dem in der ersten Stufe ein geeignet substituiertes Benzylcyanidderivat 2,

- bei der Herstellung von BIII 277 beispielsweise m-Methoxybenzylcyanid -, in dem $R_2$ Wasserstoff, $C_1$-$C_6$-Alkyl, Halogen, Hydroxy, $C_1$-$C_8$-Alkoxy, einen über ein Sauerstoff gebundenen Benzoylrest oder einen Alkylcarboxylrest mit einem geradkettigen oder verzweigten Niederalkylrest mit 1 bis 6 Kohlenstoffatomen - wobei der Alkylrest gegebenenfalls mit einem oder mehreren Halogenatom(en) substituiert sein kann, bedeutet, mit einem Bromisobuttersäureester vom Typ 3 in dem $R_3$ $C_1$-$C_8$-Alkyl oder Benzyl und bevorzugt $C_1$-$C_6$-Alkyl bedeutet und besonders bevorzugt mit Bromisobuttersäureethylester ($R_3$=$C_2H_5$) - zum entsprechend substituierten 3-Amino-2,2-dimethylbutansäureesterderivat 4 - bei der Herstellung von BIII 277: 3-Amino-4-(3-methoxyphenyl)-2,2-dimethylbutansäureethylester - umgesetzt wird:

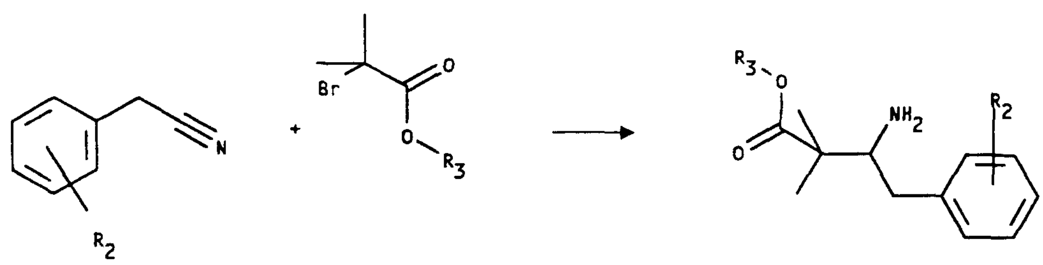

2 3 4

So ermöglicht das erfindungsgemäß vorgeschlagene Verfahren den Aufbau der aus der DE OS 20 27 077 bekannten 3-Aminodimethylbutansäure-Vorstufe in einem Reaktionschritt - ausgehend von wohlfeilen Ausgangsmaterialien -, wofür im genannten Stand der Technik 4 Stufen erforderlich sind.

Zur Durchführung dieser Umsetzung vom Typ einer Reformatsky-Reaktion wird ein Alkylhalogensilan - vorzugsweise ein Trialkylchlorsilan, besonders bevorzugt Trimethylchlorsilan - und Zinkpulver in einem unter den gewählten Reaktionsbedingungen inerten Lösungsmittel - vorzugsweise einem Ether oder in einem Halogenkohlenwasserstoff, besonders bevorzugt Dichlormethan - vorgelegt. Nach dem Verdünnen dieser Mischung mit einem polaren - inerten - Lösungsmittel, vorzugsweise einem cyclischen Ether, besonders bevorzugt Tetrahydrofuran - wird die Reaktionsmischung - vorzugsweise auf Rückflußtemperatur - erwärmt und mit einer Mischung des Bromisobuttersäureesters der allgemeinen Formel 3 mit dem geeignet substituierten Benzylcyanid der allgemeinen Formel 2 versetzt und weiter - vorzugsweise auf Rückflußtemperatur - erwärmt. Nach dem Abkühlen und dem Abfiltrieren des Zinkpulvers wird das Reaktionsgemisch mit einem hinsichtlich der Reduktion von Iminofunktionen selektiven Reduktionsmittel - vorzugsweise einem komplexen Alkaliborhydridderivat, besonders bevorzugt Natriumcyanoborhydrid - und anschließend mit einem Alkanol - vorzugsweise einem geradkettigen oder verzweigten $C_1$-$C_4$-Alkohol, besonders bevorzugt Ethanol - versetzt. Danach wird mit einer wässerigen Lösung einer basisch reagierenden Verbindung - vorzugsweise mit Ammoniaklösung, besonders bevorzugt mit konzentrierter Ammoniaklösung - versetzt und die organische Phase des Reaktionsgemisches isoliert. Nach dem Trocknen und dem Einengen i.Vak. wird der verbliebene Rückstand in einem inerten Lösungsmittel - vorzugsweise in einem aliphatischen oder aromatischen Kohlenwasserstoff, besonders bevorzugt in Toluol - aufgenommen und mit der wässerigen Lösung einer Säure - vorzugsweise einer Mineralsäure, besonders bevorzugt 2N Salzsäure - extrahiert. Abschließend wird die wässerige Phase mit der wässerigen Lösung einer basisch reagierenden Verbindung - vorzugsweise Ammoniaklösung, besonders bevorzugt mit konzentrierter Ammoniaklösung - alkalisch gestellt und danach mit einem organischen, mit Wasser nicht mischbaren Extraktionsmittel - vorzugsweise mit einem Halogenkohlenwasserstoff, besonders bevorzugt mit Dichlormethan - extrahiert. Der so erhaltene Extrakt wird nach dem Trocknen eingeengt und das 3-Amino-2,2-dimethylbutansäureesterderivat der allgemeinen Formel 4 isoliert.

Überraschenderweise wurde nun gefunden, daß auf dieser Reaktionsstufe die C-C-Kupplungsreaktion und die Reduktion der Iminogruppe zum Amin in einer Stufe durchgeführt werden kann, ohne - wie bei katalytischen Hydrierungen gemeinhin notwendig - zunächst das Imin isolieren und reinigen zu müssen. Hierdurch kann die Bildung von Hydrolyseprodukten vermieden werden, deren Auftreten bei der gewöhnlichen wässerigen Aufarbeitung zu einer Ausbeuteminderung führt.

In der zweiten Reaktionsstufe wird das so gewonnene 3-Amino-2,2-dimethylbutansäureethylesterderivat 4 mit einem Acrylsäureester ($CH_2$=CH-$COOR_4$, $R_4$ = $C_1$-$C_8$-Alkyl oder Benzyl - vorzugsweise $C_1$-$C_6$-Alkyl und besonders bevorzugt Ethyl) zu dem entsprechenden 3-(2-Ethoxycarbonylethyl)amino-2,2-dimethylbutansäureethylesterderivat 5 - bei der Herstellung von BIII 277 beispielsweise: 3-(2-Ethoxycarbonylethyl)amino-4-(3-methoxyphenyl)-2,2-dimethylbutansäureethylester ($R_2$ = $CH_3O$-)- umgesetzt:

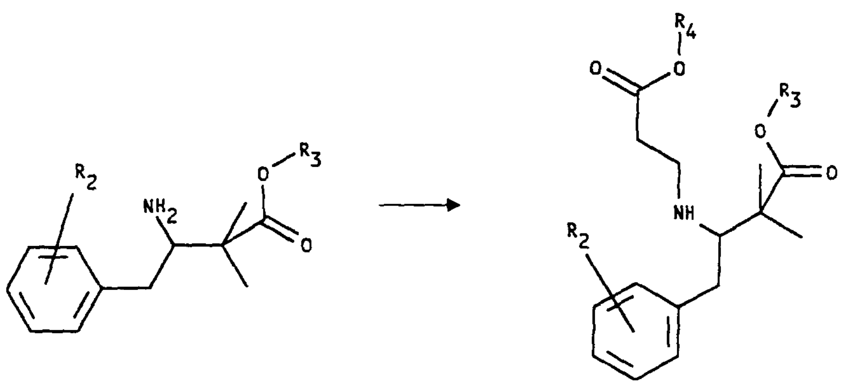

4 5

Zur Durchführung dieser Michael-Additionsreaktion wird das 3-Amino-2,2-dimethylbutansäureethylesterderivat 4 mit dem Acrylsäureester in einem unter den gewählten Reaktionsbedingungen inerten Reaktionsmedium - vorzugsweise in einem geradkettigen oder verzweigten $C_1$-$C_4$-Alkanol, besonders bevorzugt Ethanol - gelöst und - vorzugsweise auf Rückflußtemperatur - erwärmt. Nach erfolgter Reaktion wird das Lösungsmittel i.Vak. entfernt und das resultierende 3-(2-Ethoxycarbonylethyl)amino-2,2-dimethylbutansäureethylesterderivat 5 isoliert.

In der anschließenden dritten Reaktionsstufe wird das aus der vorangegangenen Reaktionsstufe resultierende 3-(2-Ethoxycarbonylethyl)amino-2,2-dimethylbutansäureesterderivat 5 - bei der Synthese von BIII 277: 3-(2-Ethoxycarbonylethyl)amino-4-(3-methoxyphenyl)-2-dimethylbutansäureester- zum entsprechenden Piperidon - im Falle der Herstellung des BIII 277: 5-Carboethoxy-3,3-dimethyl-2-(3-methoxyphenyl)methyl-4-piperidon - 6 cyclisiert:

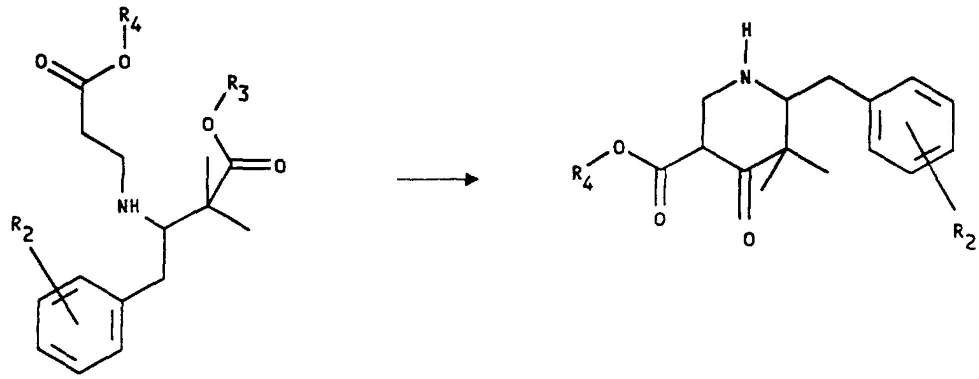

5 6

Zur Durchführung des Cyclisierungsschritts vom Typ einer Dieckmann'schen Esterkondensation wird das 3-(2-Ethoxycarbonylethyl)amino-2,2-dimethylbutansäureesterderivat 5 in einem unter den Cyclisierungsbedingungen inerten Lösungsmittel - vorzugsweise in einem aliphatischen oder aromatischen Kohlenwasserstoff, besonders bevorzugt in Toluol - gelöst und in Gegenwart einer basisch reagierenden Verbindung, vorzugsweise einem Alkalialkoholat eines verzweigten oder unverzweigten $C_1$-$C_4$-Alkohols, besonders bevorzugt Kalium-*tert.*-butylat - auf Rückflußtemperatur erhitzt und die bei diesen Temperaturen flüchtigen Bestandteile der Reaktionsmischung destillativ - beispielsweise im Rahmen einer azeotropen Reaktion - entfernt. Nach beendeter Reaktion, wird das Reaktionsgemisch hydrolysiert und mit der wässerigen Lösung einer sauer reagierenden Verbindung - vorzugsweise mit wässerigen Mineralsäuren, besonders bevorzugt mit konzentrierter Salzsäure - versetzt. Danach wird ein unter diesen Bedingungen inertes, mit Wasser nicht mischbares Extraktionsmittel - bevorzugt ein Dialkylether, besonders bevorzugt Diethylether - zugefügt und mit der wässerigen Lösung einer basisch reagierenden Verbindung, bevorzugt mit wässeriger Ammoniaklösung, besonders bevorzugt mit konzentrierter Ammoniaklösung - versetzt. Nach dem Abtrennen der organischen Phase sowie dem erschöpfenden Extrahieren der wässerigen Phase werden die vereinigten organischen Extrakte nach dem Waschen mit Wasser sowie Trocken i. Vak. eingeengt und das resultierende Piperidon vom Typ 6 - im Falle der Herstellung des BIII 277 das 5-Carboethoxy-3,3-dimethyl-2-(3-methoxyphenyl)methyl-4-piperidon - isoliert. - Alternativ kann die oben beschriebene Dieckmann-Kondensation auch mittels Titantetrachlorid In einem halogenierten Kohlenwasserstoff - vorzugsweise Dichlormethan - durchgeführt werden [M.N. Deshmukh et al., Synth. Commun. 25 (1995) 177]

In der vierten Reaktionsstufe wird das Piperidonderivat 6 unter alkalischen oder sauren Bedingungen zu dem entsprechenden 3,3-Dimethyl-4-piperidonderivat 7 verseift und decarboxyliert. Dabei richtet sich die Wahl der Reaktionsbedingungen nach der chemischen Natur des Ausgangsstoffes; so wird beispielsweise im Falle der Herstellung des BIII 277 unter den Bedingungen einer alkalischen Verseifung gearbeitet, woraus das 2-(3-Methoxyphenyl)methyl-3,3-dimethyl-4-piperidon resultiert, welches in Form eines Säureadditionsalzes - bevorzugt in Form seines Hydrohalogenids - isoliert werden kann:

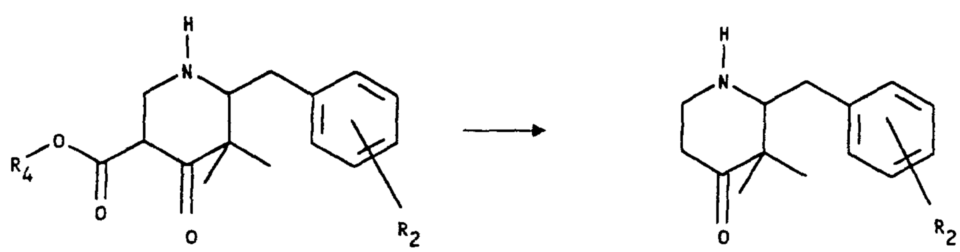

6 7

Dazu wird das Piperidonesterderivat 6 in einem polaren, wasserhaltigen Lösungsmittel bzw. Lösungsmittelgemisch - bevorzugt in einer Mischung aus einem geradkettigen oder verzweigten $C_1$-$C_4$-Alkanol und Wasser, beson-

ders bevorzugt in einem Ethanol/Wasser-Gemisch- mit einer basisch oder sauer reagierenden Verbindung - bevorzugt mit einem Alkalihydroxyd oder einer Mineralsäure, besonders bevorzugt mit Natriumhydroxyd oder im Falle des Einsatzes einer Säure beispielsweise in Gegenwart von Salzsäure oder Schwefelsäure - erhitzt; bevorzugt wird auf Rückflußtemperatur erwärmt. Nach erfolgter Verseifung wird das Reaktionsmedium i. Vak. entfernt und der Rückstand in einem für die anschließende Salzbildung geeigneten Lösungsmittel - bevorzugt einem polaren organischen Lösungsmittel, besonders bevorzugt in Aceton - aufgenommen und das Säureadditionssalz gefällt.

Die anschließende Aufspaltung des so erhaltenen Gemisches der enantiomeren 3,3-Dimethyl-4-piperidone - im Fall des BIII 277 des 2-(3-Methoxyphenyl)-methyl-3,3-dimethyl-4-piperidon-hydrochlorids - vom Typ 7 gelingt auf den bekannten Wegen zur Enantiomerentrennung - beispielsweise durch Umsetzung mit Äpfelsäure, Weinsäure, Mandelsäure oder Campfersulfonsäure, worunter Weinsäure bevorzugt wird:

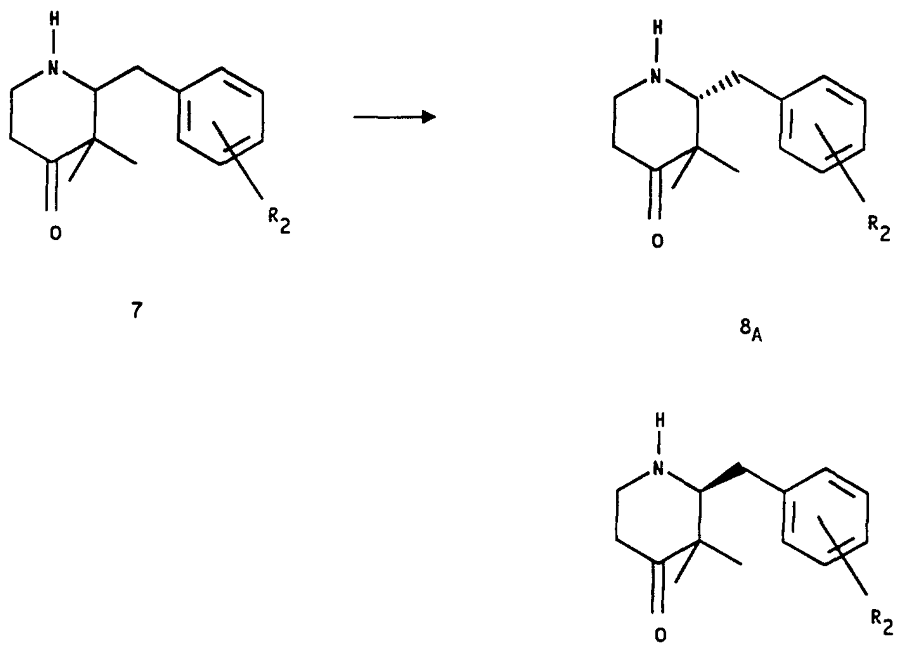

7

$8_A$

$8_B$

So liefert beispielsweise die Umsetzung mit D-(-)-Weinsäure das entsprechende enantiomerenreine 3,3-Dimethyl-4-piperidonderivat vom Typ 8A oder 8B in Form seines Hydrogentartrates, im Falle des BIII 277 z. B. das (+)-2-(3-Methoxyphenyl)methyl-3,3-dimethyl-4-piperidonium-Hydrogentartrat ($R_2$ = meta-Methoxy).

Zur Isomerentrennung - beispielsweise über die entsprechenden Tartrate - wird das Piperidonderivat 7 in Form seines Säureadditionssalzes - beispielsweise als Hydrochlorid - in Wasser gelöst und mit einer basisch reagierenden Verbindung oder - vorzugsweise - deren wässerigen Lösung versetzt; besonders bevorzugt wird konzentrierte wässerige Ammoniaklösung eingesetzt. Die wässerige Phase wird mit einem organischen, mit Wasser nicht mischbaren Lösungsmittel - bevorzugt mit einem Halogenalkan, besonders bevorzugt Dichlormethan - extrahiert. Nach dem Trocknen und dem Einengen i. Vak. wird der Rückstand in einem unter den für die Salzbildung angewandten Reaktionsbedingungen inerten Reaktionsmedium, bevorzugt in einem verzweigten oder unverzweigten $C_1$-$C_4$-Alkanol, besonders bevorzugt in Ethanol - gelöst und mit dem geeigneten Stereoisomeren einer der genannten Säuren - beispielsweise D-(-)-Weinsäure - versetzt. Gegebenenfalls wird eine ausreichende Menge eines Nichtlösemittels bezüglich des gewünschten Salzes - vorzugsweise des entsprechenden Hydrogentartrates, vorzugsweise ein verzweigtes oder unverzweigtes $C_3$-$C_8$-Alkanol, besonders bevorzugt Isopropanol - zugefügt, worauf das enantiomerenreine Isomere des Piperidons als PiperidoniumHydrogentartrat kristallisiert; das ist bei der der Herstellung des BIII 277das entsprechende (+)-2-(3-Methoxyphenyl)methyl-3,3-dimethyl-4-piperidoniumhydrogentartrat ($R_2$ = meta-Methoxy).

Überraschenderweise wurde nun festgestellt, daß nach dem Erhitzen der überwiegend das andere Enantiomere enthaltenden Mutterlauge bei einem neuen Kristallisationsversuch unter analogen Bedingungen erneut ein großer Anteil des gewünschten Enantiomeren - beispielsweise in Form seines Hydrogentartrats - gewonnen werden kann. Die thermische Racemisierung des nicht gewünschten Enantiomeren und.die anschließende Gewinnung des gewünschten Stereoisomeren kann durchaus mehrmals durchgeführt werden. Auf diese Weise kann im Falle des (+)-2-(3-Methoxyphenyl)methyl-3,3-dimethyl-4-piperidoniumhydrogentartrats die Gesamtausbeute an dem gewünschten Isomeren auf über 75 % gesteigert werden.

Die nachfolgende Wittig-Reaktion mit Methyltriphenylphosphoniumbromid führt auf der nächsten Stufe zum entsprechenden 4-Methylen-piperidinderivat 9 - im Falle des BIII 277 das (+)-2-(3-Methoxyphenyl)methyl-3,3-dimethyl-4-methylen-piperidin ($R_2$ = meta-Methoxy) - das in Form seines Säureadditionssalzes - vorzugsweise in Form eines Hydrohalogenids, besonders bevorzugt in Form seines Hydrochlorids - isoliert werden kann.

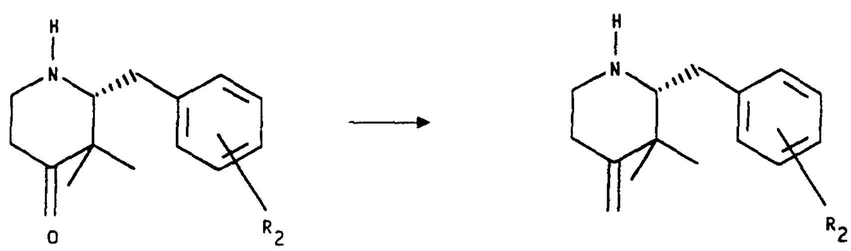

8 9

Zur Durchführung der Wittig-Reaktion wird das 3,3-Dimethylpiperidonderivat 8 in Form seines Säureadditionssalzes - beispielsweise als Hydrochlorid - in Wasser gelöst und mit einer basisch reagierenden Verbindung oder - vorzugsweise - deren wässerigen Lösung versetzt; besonders bevorzugt wird konzentrierte wässerige Ammoniaklösung eingesetzt. Die wässerige Phase wird einem organischen, mit Wasser nicht mischbaren Lösungsmittel - bevorzugt mit einem Halogenalkan, besonders bevorzugt mit Dichlormethan - extrahiert. Nach dem Trocknen und dem Einengen i. Vak. wird der Rückstand in einem unter den für die Wittig-Reaktion angewandten Reaktionsbedingungen inerten Reaktionsmedium - bevorzugt in einem cyclischen Ether, besonders bevorzugt in Tetrahydrofuran - aufgenommen und mit einem eine Methylengruppe generierenden Wittig-Reagenz - bevorzugt einem Methyltriphenylphosphoniumhalogenid, besonders bevorzugt mit Methyltriphenylphosphoniumbromid - in Gegenwart einer basisch reagierenden Verbindung, bevorzugt einem Akalialkoholat, besonders bevorzugt Kalium-*tert.*-butanolat versetzt und - in Abhängigkeit von der Reaktionsfähigkeit der jeweils eingesetzten Edukte - bei einer Temperatur im Bereich von 0 bis 80 °C - bevorzugt in einem Bereich von 20 bis 60 °C und besonders bevorzugt bei ca. 40 °C - umgesetzt. Nach erfolgter Umsetzung wird das Reaktionsgemisch mit Wasser und einem mit Wasser nicht mischbaren organischen Lösungsmittel - bevorzugt mit einem Halogenalkan, besonders bevorzugt Dichlormethan - versetzt und die organische Phase separiert. Nach erschöpfender Extraktion der wässerigen Phase und dem Trocknen der vereinigten Extrakte wird das Extraktionsmittel entfernt, der Rückstand mit einem für die Bildung eines Säureadditionssalzes geeigeneten Lösungsmittels, bevorzugt in einem verzweigten oder unverzweigten $C_1$-$C_4$-Alkanol, besonders bevorzugt in Isopropanol, gelöst und mit einer geeigneten Säure, bevorzugt einer Mineralsäure - wie z.B. einer Halogenwasserstoffsäure, besonders bevorzugt mit konzentrierter Salzsäure - versetzt und das auskristallisierte Säureadditionssalz des Wittig-Produktes 9 isoliert.

In der anschließenden 7. Reaktionssstufe erfolgt die Formylierung des Piperidin-Stickstoffs - beispielsweise mit n-Butylformiat - woraus das entsprechende enantiomerenreine N-Formyl-3,3-dimethyl-4-methylen-piperidinderivat- bei der Herstellung des BIII 277 das entsprechende (+)-N-Formyl-2-(3-methoxyphenyl)methyl-3,3-dimethyl-4-methylen-piperidin ($R_2$ = meta-Methoxy) - vom Typ 10 resultiert:

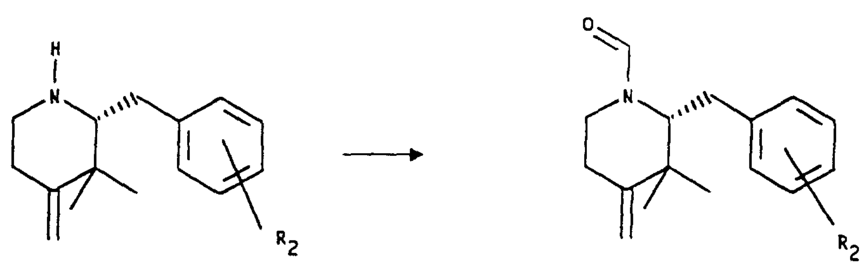

9 10

Hierzu wird das Piperidinderivat vom Typ 9, das auf der vorangegangenen Stufe als Hydrohalogenid isolierte wurde, zunächst in die entsprechende freie Base überführt, indem beispielsweise das Piperidinderivat 9 in Form seines

Hydrohalogenids in Wasser gelöst wird und mit einer basisch reagierenden Verbindung - vorzugsweise mit der wässerigen Lösung einer basisch reagierenden Verbindung und besonders bevorzugt mit konzentrierter Ammoniaklösung - versetzt, das freie Piperidin mit einem organischen Lösungsmittel, bevorzugt mit einem halogenierten Kohlenwasserstoff und besonders bevorzugt mit Dichlormethan, extrahiert. Nach dem Trocknen des Extraktes und dem Abdestillieren des Extraktionsmittels wird die freie Base in einem organischen Lösungsmittel - wie z.B. einem Kohlenwasserstoff, bevorzugt in einem Alkylaromaten, besonders bevorzugt in Toluol - aufgenommen und mit einem Formylierungsmittel - bevorzugt mit einem Alkylformiat, besonders bevorzugt mit n-Butylformiat - zur Reaktion gebracht und das Reaktionsprodukt isoliert.

In der darauf folgenden Cyclisierungsreaktion erfolgt in Gegenwart entsprechend reaktiver Lewis-Säuren - wie beipielsweise Mineralsalzsäuren, insbesondere Bromwasserstoffsäure und bevorzugt mit Sulfonsäuren und besonders bevorzugt in Gegenwart von Aluminium(III)halogeniden, ganz besonders bevorzugt in Gegenwart von Aluminiumtrichlorid - auf der achten Reaktionsstufe letztendlich der Aufbau des Benzomorphangerüsts, der im Fall der Herstellung von BIII 277 zum entsprechenden (-)-2-Formyl-3'-methoxy-5,9,9-trimethyl-6,7-benzomorphan (11) ($R_2$=m-$OCH_3$) führt.

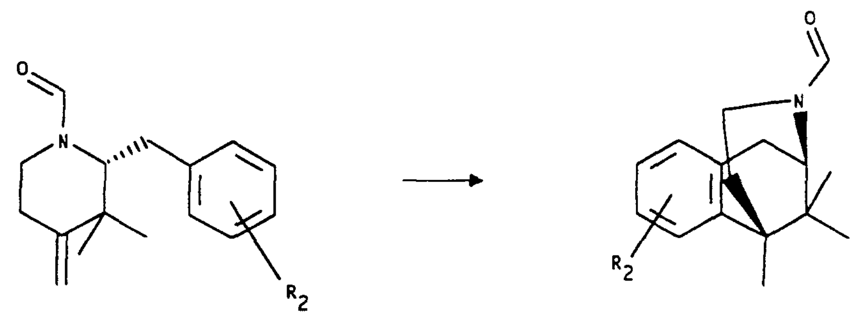

10 11

Hierzu wird das Piperidinderivat 10 einer Suspension der erwähnten Lewis-Säure - beispielsweise in Gegenwart von von Aluminium(III)chlorid - in einem unter den gewählten Reaktionsbedingungen inerten Lösungsmittel - vorzugsweise in einem halogenierten Kohlenwasserstoff, besonders bevorzugt in Dichlormethan - zugefügt. Nach dem Abschluß der Cyclisierungsreaktion wird die Reaktionsmischung vorsichtig hydrolysiert. Danach wird die wässerige Phase abgetrennt und extrahiert. Die vereinigten organischen Phasen werden nach dem Trocknen eingeengt, und das Benzomorphanderivat vom Typ 11 isoliert.

Überraschenderweise wurde gefunden, daß bei der Durchführung der Cyclisierungsreaktion - im Gegensatz zu den im Stand der Technik bereits etablierten Verfahren - unter Verwendung von $AlCl_3$ das Cylisierungsprodukt in fast quantitativer Ausbeute erhalten wird. - Im Falle der meta-Substitution des Phenylsystems weist das erfindungsgemäße Verfahren ferner denjenigen Vorteil auf, daß die Cyclisierung selektiv in der para-Position - bezogen auf die Stellung von $R_2$ - stattfindet.

Die sich anschließende neunte Reaktionsstufe resultiert in der Abspaltung der Formylgruppe und führt somit zum entsprechenden 3'-Methoxy-5,9,9-trimethyl-6,7-benzomorphan 12.

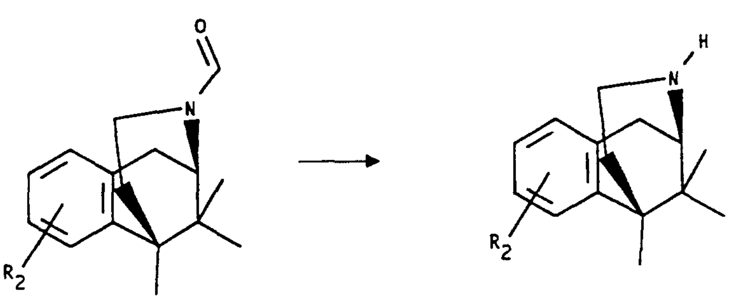

11 12

Hierzu wird das Formylbenzomorphan 11 in einem polaren Lösungsmittel - bevorzugt in einem Alkanol, besonders bevorzugt in n-Propanol - gelöst und mit einer sauer reagierenden Verbindung - bevorzugt mit der wässerigen Lösung einer Mineralsäure, besonders bevorzugt mit konzentrierter Salzsäure - versetzt und anschließend erwärmt. Nach erfolgter Abspaltung der Formylgruppe wird die Reaktionsmischung nach dem Einengen mit Wasser versetzt und mit einem mit Wasser nicht mischbaren Lösungsmittel - bevorzugt mit einem Ester einer Carbonsäure, besonders bevorzugt Essigsäureethylester - extrahiert. Die so gereinigte wässerige Phase wird bevorzugt mit konzentrierter Ammoniaklösung basisch gestellt und mit einem organischen Lösungsmittel - bevorzugt mit einem Halogenkohlenwasserstoff, besonders bevorzugt mit Dichlormethan - extrahiert. Nach dem Trocknen und Einengen der vereinigten organischen Extrakte kann auf diese Weise zum Beispiel das entsprechende (-)-3'-Methoxy-5,9,9-trimethyl-6,7-benzomorphan ($R_2$= m-$CH_3O$) isoliert werden.

Auf dieser Stufe kann jetzt - falls gewünscht - eine chemische Modifizierung des Substituenten ($R_2$) an der Phenylstruktur erfolgen - erfolgt sie nicht, erlangt $R_2$ die Bedeutung von $R_1$), wie in den Ansprüchen angegeben. So kann das aus der vorangegangenen Reaktionsstufe resultierende Benzomorphan-Derivat 12 in der anschließenden Synthesestufe unter sauren Bedingungen - vorzugsweise mit einer Mineralsäure, wie zum Beispiel mit Halogenwasserstoffsäure und besonders bevorzugt mit Bromwasserstoffsäure - einer Etherspaltung unterworfen werden, woraus die entsprechende freie Phenolteilstruktur resultiert.

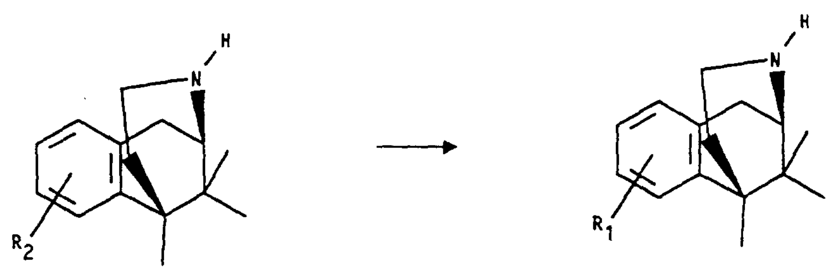

12 13

Die Etherspaltung erfolgt dabei unter sauren Bedingungen - wobei sich die Verwendung von Mineralsäuren als vorteilhaft erwiesen hat. Besonders vorteilhaft hat sich - im Falle des (-)-3'-Methoxy-5,9,9-trimethyl-6,7-benzomorphans - die Verwendung von Bromwasserstoffsäure herausgestellt. Das aus dieser Verseifungsreaktion resultierende Verseifungsprodukt kann auf diese Weise in Form seines Hydrobromids [(-)-3'-Hydroxy-5,9,9-trimethyl-6,7-benzomorphan-hydrobromid] in kristalliner Modifikation gewonnen werden.

__Beispiele__

**1. Reaktionsstufe**

3-Amino-4(3-methoxyphenyl)-2-dimethylbutansäureethylester (4) [$R_2$ = m-$CH_3O$].

229,3 g (3,5 mol) Zink in 3,0 l Dichlormethan werden unter Stickstoff mit 230 ml Trimethylchlorsilan versetzt und 20 min bei Raumtemperatur gerührt. Anschließend werden 1,1 l absolutes Tetrahydrofuran hinzugefügt und auf Rückfluß erwärmt. Zu dieser Vorlage läßt man eine Mischung aus 500 g (2,6 mol) Bromisobuttersäureethylester (1) und 226,4 g (1,5 mol) m-Methoxybenzylcyanid (2) zutropfen und erwärmt anschließend noch 1,5 h unter Rückfluß. Man läßt abkühlen, dekantiert vom überschüssigem Zink ab und versetzt nach Kühlung auf ca. 10° C mit 96,7 g (1,5 mol) Natriumcyanoborhydrid. Anschließend werden 300 ml Ethanol langsam zugetropft (Gasentwicklung). Man läßt 20 min nachreagieren, versetzt mit 1,0 l konz. Ammoniaklösung, trennt die Phasen und wäscht die organische Phase noch einmal mit einer Mischung aus 500 ml konz. Ammoniaklösung und 500 ml Wasser. Die organische Phase wird über Natriumsulfat getrocknet und im Vakuum eingeengt. Der Rückstand wird in 2,3 l Toluol aufgenommen und zweimal mit je 1,8 l 2 n Salzsäure ausgeschüttelt. Anschließend wird die Wasserphase mit 700 ml konz. Ammoniaklösung alkalisch gestellt und zweimal mit 2,2 l Dichlormethan extrahiert. Nach dem Trocknen der organischen Phase über Natriumsulfat wird im Vakuum eingeengt. Man isoliert den 3-Amino-4-(3-methoxyphenyl)-2--dimethylbutansäureethylester (4) in einer Ausbeute von 322,5 g (81 % d.Th.) als gelbes Öl.

**2. Reaktionsstufe:**

3-(2-Ethoxycarbonylethyl)amino-4-(3-methoxyphenyl)-2-dimethylbutansäureethylester (5) [$R_2$ = m-$CH_3O$]

382,2 g (1,4 mol) 3-Amino-4-(3-methoxyphenyl)-2-dimethylbutansäureethylester (4) und 195,4 ml (1,8 mol) Acrylsäureethylester werden in 570 ml abs. Ethanol gelöst und 7 d unter Rückfluß erhitzt. Anschließend wird der Ansatz im Vakuum vollständig eingeengt. Man isoliert den 3-(2-Ethoxycarbonylethylamino-4-(3-methoxyphenyl)-dimethylbutansäureethylester (5) in einer Ausbeute von 469,2 g (89,2 % d.Th.) als rotbraunes Öl.

**3. Reaktionsstufe:**

5-Carboethoxy-3,3-dimethyl-2-(3-methoxyphenyl)methyl-4-piperidon (6) [$R_2$ = m-$CH_3O$]

469,2 g (1,3 mol) 3-(2-Etoxycarbonylethyl)amino-4-(3-methoxyphenyl)-2-dimethylbutansäureethylester (5) [$R_2$ = m-$CH_3O$] werden in 7,8 l Toluol gelöst und zunächst ca. 100 ml eines Lösungsmittel/Wasser-Gemisches abgeschleppt. Man läßt auf ca. 70°C abkühlen, versetzt mit 158,3 g (1,4 mol) Kalium-*tert*.-butylat und erwärmt 40 min auf 105° C, wobei das sich bildende Ethanol abdestilliert wird. Anschließend kühlt man auf 5° C ab und vesetzt mit 1,2 l Eiswasser und 280 ml konz. Salzsäure. Man versetzt mit 1,2 l Ether und 220 ml konz. Ammoniaklösung, trennt die organische Phase ab und extrahiert die wässerige Phase noch zweimal mit je 600 ml Diethylether. Die vereinigten organischen Phase werden zweimal mit je 600 ml Wasser gewaschen, über Natriumsulfat getrocknet und i.Vak. eingeengt. Man isoliert das 5-Carbethoxy-3,3-dimethyl-2-(3-methoxyphenyl)-methyl-4-piperidon (6) in einer Ausbeute von 390,1 g (95,1 % d. Th.) als rotbraunes Öl.

**4. Reaktionsstufe:**

2-(3-Methoxyphenyl)methyl-3,3-dimethyl-4-piperidon-hydrochlorid (7) [$R_2$ = m-$CH_3O$]

390,1 g (1,22 mol) 5-Carboethoxy-3,3-dimethyl-2-(3-methoxyphenyl)methyl-4-piperidon (6) [$R_2$ = m-$CH_3O$] werden in einer Mischung aus 204,8 g (5,1 mol) Natriumhydroxyd, 680 ml Ethanol und 680 ml Wasser gelöst und 20 min unter Rückfluß erhitzt. Man zieht das Lösungsmittel i. Vak. ab, nimmt den Rückstand in Aceton auf und fällt das Hydrochlorid mit etherischer Salzsäure. Man isoliert das 2-(3-Methoxyphenyl)methyl-3,3-dimethyl-4-piperidon-hydrochlorid (7) in einer Ausbeute von 311,9 g (90,1 % d.Th.) in Form weißer Kirstalle vom Schmp.: 224 - 225°C.

**5. Reaktionsstufe:**

Enantiomerentrennung des Piperidons

(+)-2-(3-Methoxyphenyl)methyl-3,3,-dimethyl-4-piperidoniumhydrogentartrat (8) [$R_2$ = m-$CH_3O$]

28,7 g (101 mmol) 2-(3-Methoxyphenyl)methyl-3,3,dimethyl-4-piperidon-hydrochlorid (7) werden in 57 ml Wasser gelöst. Die wässerige Phase wird dreimal mit je 35 ml Dichlormethan extrahiert. Die vereinigten organischen Phasen werden mit 25 ml Wasser gewaschen, anschließend mit Natriumsulfat getrocknet und das Lösungsmittel danach im Vakuum abgezogen. Der Rückstand wird bei 80° C im Vakuum bis zur Gewichtskonstanz getrocknet (24,7 g). Anschließend wird der Rückstand in 200 ml Ethanol mit 15 g (100 mmol) D-(-)-Weinsäure warm gelöst und unter Rühren werden 50 ml Isopropanol und eine kleine Menge Impfkristalle zugefügt. Man läßt 24 h bei Raumtemperatur kristallisieren und saugt von den Kristallen ab (15 g, Schmp.: 142° C; $[\alpha]_D^{25}$ = + 31,7° (c=1 in MeOH)). Die Mutterlauge wird im Vakuum bis zur Trockne eingedampft, mit 150 ml einer Mischung aus Ethanol und Isopropanol (80 : 20) versetzt und 20 h unter Rückfluß erhitzt. Danach wird die Lösung wieder mit einer geringen Menge Impfkristalle versetzt und 6 Tage stehen gelassen. Anschließend wird erneut abgesaugt (6,65 g, Schmp. 142°C; $[\alpha]_D^{25}$ = + 32,2° (C=1 in Methanol )) und die Mutterlauge weitere 20 h unter Rückfluß erhitzt und anschließend bis zur Trockenheit eingedampft. Der Rückstand wird in 100 ml Wasser aufgenommen, 10 ml 2N Salzsäure zugefügt und dreimal mit je 25 ml Diethylether extrahiert. Die etherische Phase wird verworfen (nichtbasische Verunreinigungen) und die wässerige Phase mit konz. Ammoniaklösung alkalisch gestellt und erneut dreimal mit je 30 ml Diethylether extrahiert. Die vereinigten etherischen Phasen werden über Magnesiumsulfat getrocknet und im Vakuum eingeengt (10,35 g Rückstand). Der Rückstand wird zusammen mit 6,28 g (42 mmol) D-(+)-Weinsäure in 104 ml einer Mischung aus Ethanol und Isopropanol (80 : 20) warm gelöst. Man versetzt mit Impfkristallen und läßt 1 d bei Raumtemperatur kristallisieren. Die Kristalle werden abgesaugt (5,8 g, Schmp.: 142°C; $[\alpha]_D^{25}$ = + 31,6° (c=1 in Methanol)). Die Mutterlauge wird eingedampft und der Rückstand (11,5 g) in 72 ml einer Mischung aus Ethanol und Isopropanol (80 : 20) gelöst und 20 h unter Rückfluß erhitzt. Anschließend

versetzt man mit Impfkristallen und läßt 6 d bei Raumtemperatur stehen. Die ausgefallenen Kristalle werden abgesaugt (2,66 g Schmp.: 140° C; $[\alpha]_D^{25}$ = + 31,8° (c=1 in Methanol) und mit den vorherigen Fraktionen vereinigt. Auf diese Weise gewinnt man das (+)-2-(3-Methoxyphenyl)methyl-3,3-dimethyl-4-piperidoniumhydrogentartrat (8) in einer Gesamtausbeute von 30,11 g (75 % d.Th.).

**6. Reaktionsstufe:**

(+)-2-(3-Methoxyphenyl)methyl-3,3-dimethyl-4-methylen-piperidinhydrochlorid (9)

24,0 g (60,3 mmol) (+)-2-(3-Methoxyphenyl)methyl-3,3-dimethyl-4--piperidoniumhydrogentartrat (8) werden in 50 ml Wasser gelöst und mit 15 ml konz. Ammoniaklösung und 50 ml Dichlormethan versetzt. Man trennt die Phasen, extrahiert die wässerige Phase zweimal mit 25 ml Dichlormethan und trocknet die vereinigte organische Phase über Magnesiumsulfat. Anschließend wird das Lösungsmittel im Vakuum abgezogen und der Rückstand in 30 ml abs. Tetrahydrofuran aufgenommen.

25,7 g (720 mmol) Methyltriphenylphosphoniumbromid werden in 205 ml absolutem Tetrahydrofuran suspendiert und unter Sticksstoff mit 8,1 g (720 mmol) Kalium-*tert*.-butylat bei Raumtemperatur versetzt. Man läßt 30 min. bei 40° C rühren, kühlt wieder auf Raumtemperatur ab und versetzt innerhalb von 10 min. mit der oben hergestellten Lösung des Piperidons in 30 ml Tetrahydrofuran. Man läßt 1 h bei Raumtemperatur nachreagieren, kühlt auf 10 °C und versetzt innerhalb von 15 min mit 66 ml Wasser. Anschließend wird das Tetrahydrofuran im Vakuum abgezogen, der Rückstand mit 46 ml Dichlormethan und 30 ml Eiswasser versetzt. Die Phasen werden getrennt, die wässerige Phase noch zweimal mit je 15 ml Dichlormethan extrahiert und die vereinigten organischen Extrakte noch einmal mit 40 ml Waser ausgeschüttelt. Anschließend wird über Magnesiumsulfat getrocknet, das Lösungsmittel im Vakuum abgezogen, der Rückstand in 85 ml Isopropanol gelöst und unter Eiskühlung mit 5,7 ml konz. Salzsäure versetzt. Nach 1 h wird abgesaugt (8,5 g), die Mutterlauge zur erneuten Kristallisation mit 150 ml Diethylether versetzt und nach 1 h erneut abgesaugt (5,2 g). Die Mutterlauge wird i. Vakuum eingedampft, der Rückstand wieder in 30 ml Isopropanol aufgenommen und mit 200 ml Diethylether versetzt. Nach dreistündigem Kristallisieren bei Raumtemperatur wird abgesaugt (2,1 g) und anschließend alle Kristallisationsfraktionen bei 60° C getrocknet. Alle drei Fraktionen erweisen sich im Dünnschichtchromatogramm (Dichlormethan: Methanol: konz. Ammoniak = 95 : 5 : 0,1 ) identisch.

Man isoliert auf diese Weise das (+)-2-(3-Methoxyphenyl)-methyl-3,3-dimethyl-4--methylen-piperidin (9) in Form seines Hydrochlorids in einer Ausbeute von 15,8 g (93,2% d. Th.) vom Schmp.: 199 - 200° C; $[\alpha]_D^{25}$ = + 59,9 (c=1 in Methanol).

**7. Reaktionsstufe:**

(+)-N-Formyl-2-(3-methoxyphenyl)methyl-3,3-dimethyl-4-methylen-piperidin (10) [$R_2$ = 3-$CH_3O$)]

12,7 g (45 mmol) (+)-2-(3-Methoxyphenyl)methyl-3,3,-dimethyl-4-methylen-piperidin-hydrochlorid (9) werden in 50 ml Wasser gelöst und mit 8 ml konz. Ammoniak versetzt. Man extrahiert dreimal mit je 20 ml Dichlormethan, trocknet über Magnesiumsulfat und zieht das Lösungsmittel i. Vakuum ab. Der Rückstand wird in 15 ml Toluol aufgenommen und erneut eingedampft, erneut in 75 ml Toluol aufgenommen und mit 23,1 g (22g mmol) n-Butylformiat 4 h unter Rückfluß erhitzt. Anschließend wird i. Vakuum eingedampft, wonach 12,2 g (99,5 % d. Th.) des (+)-N--Formyl-2-(3-methoxyphenyl)methyl-3,3-dimethyl-4-methylenpiperidins (10) als Öl verbleiben $[\alpha]_D^{25}$ = + 52,0° (c=1 in Methanol).

**8. Reaktionsstufe:**

(-)-2-Formyl-3'-methoxy-5,9,9-trimethyl-6,7-benzomorphan (11) [$R_2$ = 3'-$CH_3O$]

Man legt 16 g (120 mmol) Aluminiumchlorid in 140 ml Dichlormethan bei einer Temperatur von -10°C vor und tropft 10,9 g (40 mmol) (+)-N-Formyl-2-(3-methoxyphenyl)methyl-3,3,-dimethyl-4-methylenpiperidin- gelöst in 35 ml Dichlormethan - so langsam zu, daß die Temperatur nicht über -5° steigt (ca. 45 min.). Anschließend läßt man 30 min bei 0°C nachreagieren, gießt auf 100 g Eis und läßt kräftig rühren. Die organische Phase wird abgetrennt, die wässerige Phase noch zweimal mit je 30 ml Dichlormethan extrahiert, die vereinigten organischen Extrakte getrocknet und das Lösungsmittel im Vakuum abgezogen.

Man erhält so das (-)-2-Formyl-3'-methoxy-5,9,9-trimethyl-6,7-benzomorphan (11) in einer Ausbeute von 10,9 g (99,6 % d.Th. als Öl; $[\alpha]_D^{25}$ = - 198,4° (c=1 in Methanol)).

**9. Reaktionsstufe:**

(-)-3'-Methoxy-5,9,9-trimethyl-6,7-benzomorphan (12) [$R_2$ = 3'-$CH_3O$]

9,57 g (35 mmol) (-)-2-Formyl-3'-methoxy-5,9,9-trimethyl-6,7-benzomorphan (11) werden in 75 ml n-Propanol gelöst und mit 25 ml konz. Salzsäure und 14,3 ml Wasser 14 h unter Rückfluß erhitzt. Anschließend wird i. Vakkum eingedampft, der Rückstand in 50 ml Eiswasser aufgenommen und dreimal mit je 20 ml Essigsäureethylester extrahiert (wird verworfen). Die wässerige Phase wird mit 55 ml konz. Ammoniak versetzt und dreimal mit 25 ml Dichlormethan extrahiert. Die vereinigten organischen Extrakte werden über Magnesiumsulfat getrocknet und im Vakuum eingeengt. Man isoliert auf diese Weise das (-)-3'-Methoxy-5,9,9-trimethyl-6,7-benzomorphan (11) in einer Ausbeute von 7,9 g (92,0 % d. Th.) als Öl; $[\alpha]_D^{25}$ =-66,0° (c=1 in Methanol).

**10. Reaktionsstufe:**

(-)-3'-Hydroxy-5,9,9-trimethyl-6,7-benzomorphan-hydrobromid (13) [$R_1$ = 3'-OH]

10 g (41 mmol) (-)-3'-Methoxy-5,9,9-trimethyl-6,7-benzomorphan (12) werden mit 22,5 ml Wasser und 77,5 ml 62 %iger Bromwasserstoffsäure 2 h unter Rückfluß erhitzt. Anschließend wird im Vakuum eingeengt und der Rückstand aus ca. 80 ml Aceton umkristallisiert, wonach 11,8 g (92,8 %) d.Th.) des (-)-3'-Hydroxy-5,9,9-trimethyl-6,7-benzomorphan-hydrobromids (13) in Form von Kristallen vom Schmp.: >290° C; $[\alpha]_D^{25}$ =- 55,8° (c=1 in Methanol) erhalten werden.

**Patentansprüche**

1. Verfahren zur Herstellung von Norbenzomorphanen der allgemeinen Formel 1

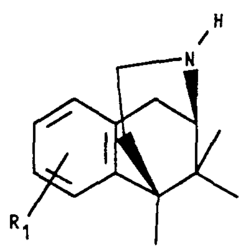

1

worin

$R_1$ Wasserstoff, $C_1$-$C_6$-Alkyl, Halogen, Hydroxy, $C_1$-$C_8$-Alkoxy, einen über ein Sauerstoff gebundenen Benzoylrest oder einen Alkylcarboxylrest mit einem geradkettigen oder verzweigten Niederalkylrest mit 1 bis 6 Kohlenstoffatomen - wobei der Alkylrest gegebenenfalls mit einem oder mehreren Halogenatom(en), die untereinander gleich oder verschieden sein können, substituiert sein kann -
Nitro, Cyano, -$NH_2$, -NH($C_1$-$C_8$-Alkyl), -N($C_1$-$C_8$-Alkyl)$_2$, wobei die Alkylreste gleich oder verschieden sein können, -NH-Acyl-($C_1$-$C_8$-Alkyl), worin Acyl für Benzoyl oder einen Alkylcarbonylrest mit einem geradkettigen oder verzweigten Niederalkylrest mit 1 bis 6 Kohlenstoffatom(en), wobei der Alkylrest gegebenenfalls mit einem oder mehreren Halogenatom(en), die untereinander gleich oder verschieden sein können, substituiert sein kann,

bedeuten kann,
dadurch gekennzeichnet, daß man

a) ein Benzylcyanid der allgemeinen Formel 2, in der $R_2$ Wasserstoff, $C_1$-$C_6$-Alkyl, Halogen, Hydroxy, $C_1$-$C_8$-Alkoxy, einen über ein Sauerstoff gebundenen Benzoylrest oder einen Alkylcarboxylrest mit einem geradkettigen oder verzweigten Niederalkylrest mit 1 bis 6 Kohlenstoffatomen - wobei der Alkylrest gegebenenfalls mit

einem oder mehreren Halogenatom (en), die untereinander gleich oder verschieden sein können, substituiert sein kann - mit einem Bromisobuttersäureeester der allgemeinen Formel 3, in der $R_3$ $C_1$-$C_8$ -Alkyl oder Benzyl bedeutet, in Gegenwart eines Alkylhalogensilans und Zinkpulver in einem inerten Lösungsmittel und in Gegenwart eines hinsichtlich der Reduktion von Iminofunktionen selektiven Reduktionsmittels den Bedingungen einer Reformatsky-Reaktion unterwirft und das resultierende 3-Amino-2,2-dimethylbutansäureethylesterderivat der allgemeinen Formel 4 isoliert

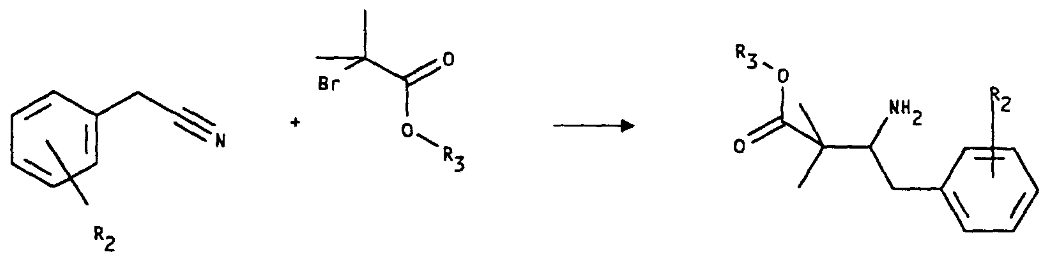

2 3 4

und

b) das 3-Amino-2,2-dimethylbutansäureesterderivat der allgemeinen Formel 4 mit einem Acrylsäureester, in dem die Alkoholkomponente $R_4$ die Bedeutung einer $C_1$-$C_8$-Alkylgruppe oder einer Benzylgruppe hat, den Bedingungen einer Michael-Additionsreaktion unterwirft und das resultierende 3-(2-Ethoxycarbonylethyl)amino-2,2-dimethylbutansäureesterderivat der allgemeinen Formel 5 isoliert

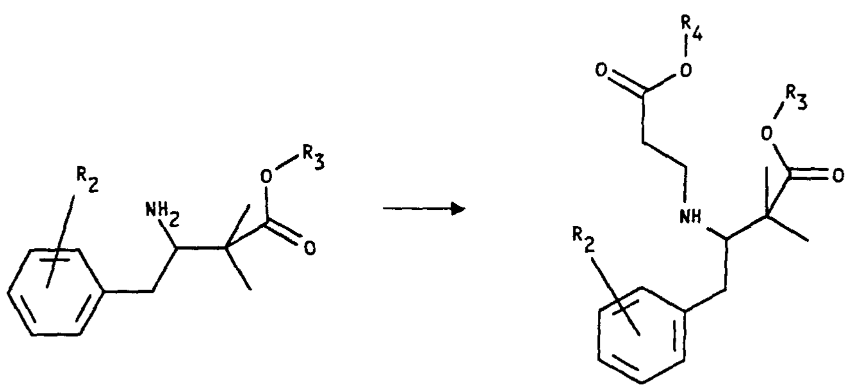

4 5

und

c) das so hergestellte 3-(2-Ethoxycarbonylethyl)amino-2,2-dimethylbutansäureesterderivat der allgemeinen Formel 5 in einem inerten Lösungsmittel in Gegenwart einer basisch reagierenden Verbindung den Bedingungen einer Dieckmann'schen Esterkondensation unterwirft und das resultierende Piperidonderivat der allgemeinen Formel 6 isoliert

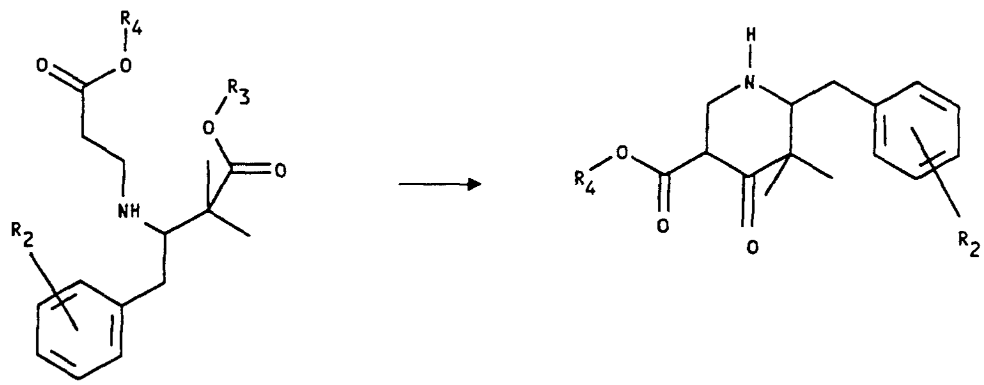

5 6

und

d) das Piperidonderivat 6 unter sauren oder alkalischen Bedingungen in einem polaren Lösungsmittel oder Lösungsmittelgemisch unter Erhitzen zum entsprechenden 3,3-Dimethylpiperidonderivat der allgemeinen Formel 7 verseift und decarboxyliert, isoliert und gegebenenfalls mit einer Säure das entsprechende Säureadditionssalz herstellt und isoliert

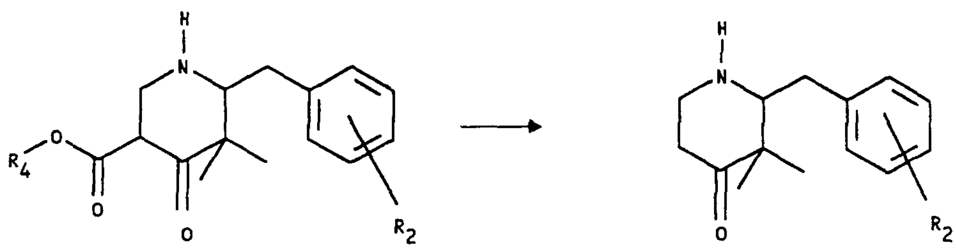

6 7

und

e) das so erhaltene Stereoisomerengemisch gegebenenfalls nach Freisetzung der jeweiligen enantiomeren freien Basen in einem - hinsichtlich der Enantiomerentrennung - inerten Reaktionsmedium löst, mit einem geeigneten Stereoisomeren einer zur Salzbildung mit einem Stereoisomeren der Enantiomerenmischung geeigneten organischen Säure versetzt, das gewünschte Stereoisomere in Form seines Additionssalzes mit der optisch aktiven Säure isoliert, die das nicht gewünschte Isomere enthaltende Mutterlauge erhitzt und dabei das nicht gewünschte Enantiomere thermisch in das gewünschte Stereoisomere überführt, mit einer optisch aktiven enantiomerenreinen, zur Bildung eines Säureadditionssalzes befähigten organischen Säure versetzt und das so als Säureadditionssalz vorliegende gewünschte Stereoisomere gegebenenfalls unter Zugabe eines sich bezüglich des gewünschten Salzes als Nichtlösemittels verhaltenden Mediums zufügt und isoliert und diesen Vorgang gegebenenfalls wiederholt

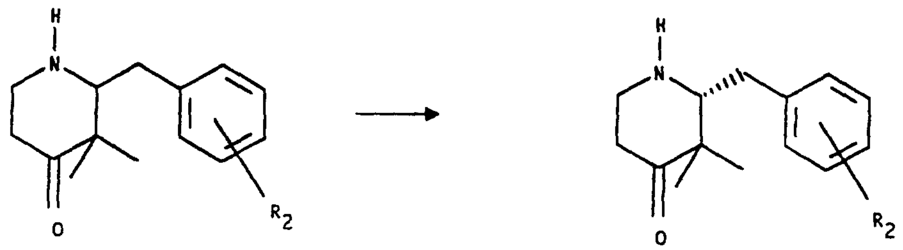

7

$8_A$

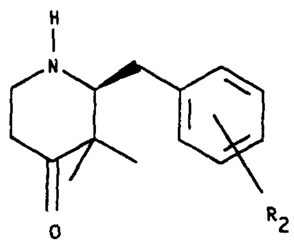

$8_B$

und

f) das so erhaltene reine Stereoisomere nach dem Freisetzen aus dem enantiomerenreinen Säureadditionssalz in einem inerten Lösungsmittel mit einem eine Methylengruppe generierenden Wittig-Reagenz in Gegenwart einer basischen reagierenden Verbindung unterwirft und das Reaktionsprodukt vom Typ 9 oder das entsprechende Stereoisomere gegebenenfalls in Form seines Säureadditionsalzes isoliert

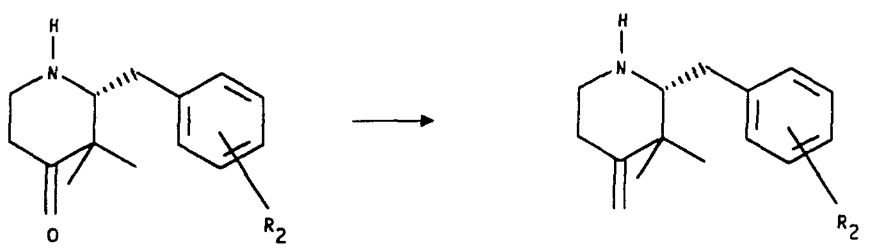

8

9

und

g) das aus der Wittig-Reaktion hervorgegangene Alken 9 gegebenenfalls zunächst aus seinem Säureadditionssalz freisetzt und die freie Base vom Typ 9 in einem organischen Lösungsmittel löst und mit einem Formylierungsmittel einer Formylierungsreaktion am Piperidinstickstoff unterwirft und das Reaktionsprodukt vom Typ 10 oder dessen entsprechende Stereoisomere isoliert

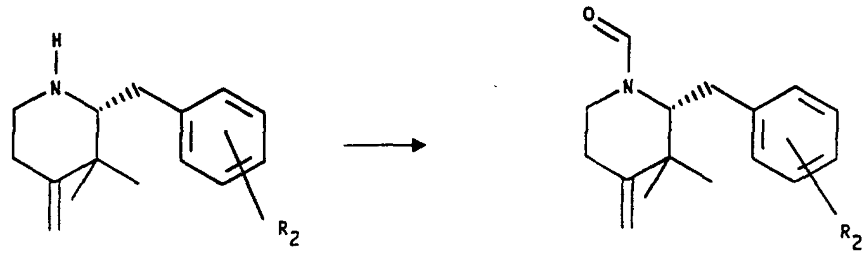

9 10

und

h) die so gewonnene Formylverbindung 10 - bzw. das entsprechende Stereoisomere - in einem inerten Lösungsmittel löst und mit einer Lewis-Säure umsetzt und das aus dieser Umsetzung resultierende Cyclisierungsprodukt vom Typ 11 isoliert

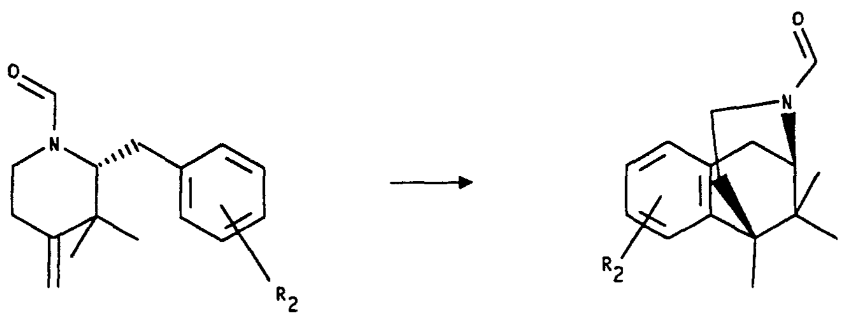

10 11

und

i) das aus der Cyclisierungsreaktion resultierende Benzomorphanderivat in einem polaren Lösungsmittel löst und mit einer sauer reagierenden Verbindung zur Reaktion bringt und das aus dieser Umsetzung resultierende deformylierte Norbenzomorphan vom Typ 12 gegebenenfalls in Form seines Säureadditionssalzes isoliert

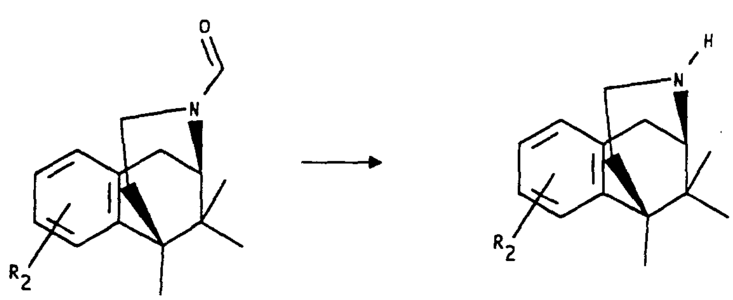

11 12

und

j) falls gewünscht nach Freisetzung der freien Benzomorphan-Base den Substituenten $R_2$ - im Falle dieser

eine Alkoxygruppe verkörpert - auf dem Wege einer Etherspaltung in eine freie Hydroxyfunktion überführt und das Reaktionsprodukt - gegebenenfalls in Form seines Säureadditionssalzes vom Typ 13, wobei die Base der allgemeien Formel 1 entspricht, isoliert.

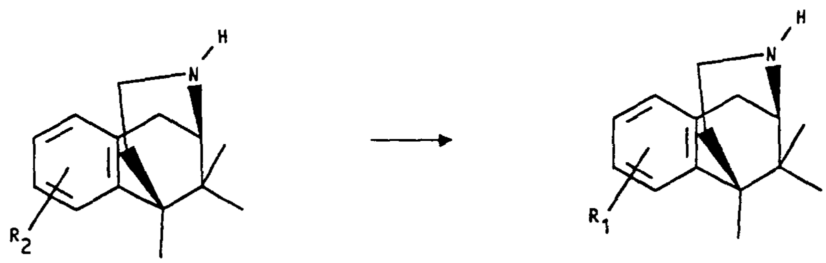

12 13

**2.** Verfahren zur Herstellung von Norbenzomorphan der allgemeinen Formel 1 nach Anspruch 1

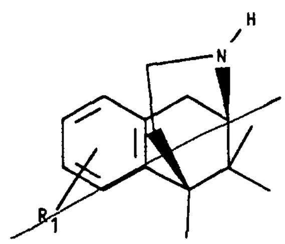

1

worin

$R_1$ die in Anspruch 1 angegebene Bedeutung hat

dadurch gekennzeichnet, daß man

a) ein Benzylcyanid der allgemeinen Formel 2, in der $R_2$ die in Anspruch 1 angegebene Bedeutung hat - mit einem Bromisobuttersäureester der allgemeinen Formel 3, in der $R_3$ $C_1$-$C_8$ -Alkyl oder Benzyl bedeutet, in Gegenwart eines Alkylhalogensilans und Zinkpulver in einem inerten Lösungsmittel umsetzt, die Reaktionsmischung erwärmt, nach erfolgter Reaktion abkühlen läßt, das Zinkpulver abtrennt und das Reaktionsgemisch mit einem hinsichtlich der Reduktion von Iminofunktionen selektiven Reduktionsmittel versetzt und das Reaktionsgemisch mit einem Alkanol verdünnt und danach mit einer wässerigen Lösung einer basisch reagierenden Verbindung versetzt, die organische Phase abtrennt und einengt, den Rückstand in einem inerten Lösungsmittel aufnimmt, die erhaltene Lösung mit der wässerigen Lösung einer Säure extrahiert, die vereinigten wässerigen Extrakte mit einer basisch reagierenden Verbindung alkalisch stellt, diese alkalische Lösung mit einem mit Wasser nicht mischbaren organischen Lösungsmittel extrahiert und das resultierende 3-Amino-2,2-dimethylbutansäureesterderivat der allgemeinen Formel 4 isoliert
und

b) das 3-Amino-2,2-dimethylbutansäureesterderivat der allgemeinen Formel 4 mit einem Acrylsäureester, in dem die Alkoholkomponente $R_4$ die Bedeutung einer $C_1$-$C_8$-Alkylgruppe oder einer Benzylgruppe hat, einer Michael-Additionsreaktion in einem inerten Lösungsmittel unterwirft, das Reaktionsmedium nach erfolgter Reaktion entfernt und das resultierende 3-(2-Ethoxycarbonylethyl)amino-2,2-dimethylbutansäureethylesterderivat der allgemeinen Formel 5 isoliert
und

c) das so hergestellte 3-(2-Ethoxycarbonylethyl)amino-2,2-dimethylbutansäureesterderivat der allgemeinen

Formel 5 in einem inerten Lösungsmittel in Gegenwart einer basisch reagierenden Verbindung den Bedingungen einer Dieckmann'schen Esterkondensation unterwirft, die flüchtigen Bestandteile der aus der Cyclisierungsreaktion resultierenden Reaktionsmischung destillativ entfernt, danach das Gemisch hydrolysiert und mit der wässerigen Lösung einer sauer reagierenden Verbindung versetzt, die resultierende Mischung mit einem mit Wasser nicht mischbaren organischen Lösungsmittel und einer wässerigen Lösung einer basisch reagierenden Verbindung versetzt, die vereinigten organischen Extrakte einengt und das resultierende Piperidonderivat der allgemeinen Formel 6 isoliert
und

d) das Piperidonderivat 6 unter sauren oder alkalischen Bedingungen in einem polaren Lösungsmittel oder Lösungsmittelgemisch unter Erhitzen zum entsprechenden 3,3-Dimethylpiperidonderivat der allgemeinen Formel 7 verseift und decarboxyliert, isoliert und gegebenenfalls mit einer Säure das entsprechende Säureadditionssalz herstellt und isoliert
und

e) das so erhaltene Stereoisomerengemisch gegebenenfalls nach Freisetzung der jeweiligen enantiomeren freien Basen in einem - hinsichtlich der Enantiomerentrennung - inerten Reaktionsmedium löst, mit einem geeigneten Stereoisomeren einer zur Salzbildung mit einem Stereoisomeren der Enantiomerenmischung geeigneten organischen Säure versetzt, das gewünschte Stereoisomere in Form seines Säureadditionssalzes mit der optisch aktiven Säure isoliert, die das nicht gewünschte Isomere enthaltende Mutterlauge erhitzt und dabei das nicht gewünschte Enantiomere thermisch in das gewünschte Stereoisomere überführt, mit einer optisch aktiven enantiomerenreinen, zur Bildung eines Säureadditionssalzes befähigten organischen Säure versetzt und das so als Säureadditionssalz vorliegende gewünschte Stereoisomere gegebenenfalls unter Zugabe eines sich bezüglich des gewünschten Salzes als Nichtlösemittels verhaltendes Medium zufügt und das Salz isoliert und gegebenenfalls diesen Vorgang wiederholt
und

f) das so erhaltene reine Stereoisomere nach dem Freisetzen aus dem enantiomerenreinen Säureadditionssalz in einem inerten Lösungsmittel mit einem eine Methylengruppe generierenden Wittig-Reagenz in Gegenwart einer basischen reagierenden Verbindung in einem Temperaturintervall von 0 bis 80 °C einer Wittig-Reaktion unterwirft, das Reaktionsgemisch nach erfolgter Umsetzung mit Wasser sowie mit einem mit Wasser nicht mischbaren organischen Lösungsmittel versetzt, die wässerige Phase erschöpfend extrahiert, das Reaktionsprodukt vom Typ 9, oder nach Zugabe einer Protonensäure, das entsprechende Stereoisomere in Form seines Säureadditionssalzes isoliert
und

g) das aus der Wittig-Reaktion hervorgegangene Alken 9 ggf. zunächst aus seinem Säureadditionssalz freisetzt und die freie Base vom Typ 9 in einem organischen Lösungsmittel löst und mit einem Formylierungsmittel einer Formylierungsreaktion am Piperidinstickstoff unterwirft und das Reaktionsprodukt vom Typ 10 oder dessen entsprechendes Stereoisomere isoliert
und

h) die so gewonnene Formylverbindung 10 - bzw. das entsprechende Stereoisomere - in einem inerten Lösungsmittel löst und mit einer Lewis-Säure umsetzt und das aus dieser Umsetzung resultierende Cyclisierungsprodukt vom Typ 11 isoliert
und

i) das aus der Cyclisierungsreaktion resultierende Benzomorphanderivat in einem polaren Lösungsmittel löst und mit einer sauer reagierenden Verbindung zur Reaktion bringt und das aus dieser Umsetzung resultierende deformylierte Norbenzomorphan vom Typ 12 nach Zugabe der Mineralsäure in Form seines Säureadditionssalzes isoliert
und

j) falls gewünscht nach Freisetzung der freien Benzomorphan-Base den Substituenten $R_2$ - im Falle dieser eine Alkoxygruppe verkörpert - auf dem Wege einer Etherspaltung in eine freie Hydroxyfunktion überführt und das Reaktionsprodukt - gegebenenfalls in Form seines Säureadditionssalzes vom Typ 13, wobei die Base der allgemeien Formel 1 entspricht, isoliert.

**3.** Verfahren zur Herstellung von Norbenzomorphan der allgemeinen Formel 1, in der $R_1$ die in Anspruch 1 angegebene Bedeutung hat, nach Anspruch 2, dadurch gekennzeichnet, daß man

a) ein Benzylcyanid der allgemeinen Formel 2, in der $R_2$ die in Anspruch 1 angegebene Bedeutung hat - mit einem Bromisobutylbuttersäureester der allgemeinen Formel 3, in der $R_3$ $C_1$-$C_6$-Alkyl bedeutet, in Gegenwart eines Trialkylhalogensilans und Zinkpulver in einem Ether und einem Halogenalkan umsetzt, die Reaktionsmischung erwärmt, nach erfolgter Reaktion abkühlen läßt, das Zinkpulver abtrennt und das Reaktionsgemisch mit einem hinsichtlich der Reduktion von Iminofunktionen selektiven komplexen Alkaliborhydridderivat versetzt und das Reaktionsgemisch mit einem $C_1$-$C_4$-Alkohol verdünnt und danach mit einer wässerigen Ammoniaklösung versetzt, die organische Phase abtrennt und einengt, den Rückstand in einem aliphatischen oder aromatischen Kohlenwasserstoff aufnimmt, die erhaltene Lösung mit der wässerigen Lösung einer Mineralsäure extrahiert, die vereinigten wässerigen Extrakte mit wässeriger Ammoniaklösung alkalisch stellt, diese alkalische Lösung mit einem Halogenkohlenwasserstoff extrahiert und das resultierende 3-Amino-2,2-dimethylbutansäureethylester-Derivat der allgemeinen Formel 4 isoliert
und

b) das 3-Amino-2,2-dimethylbutansäureesterderivat der allgemeinen Formel 4 mit einem Acrylsäureester, in dem die Alkoholkomponente $R_4$ die Bedeutung einer $C_1$-$C_8$-Alkylgruppe oder einer Benzylgruppe hat, einer Michael-Additionsreaktion in einem geradkettigen oder verzweigten $C_1$-$C_4$-Alkohol unterwirft, das Reaktionsmedium nach erfolgter Reaktion entfernt und das resultierende 3-(2-Ethoxycarbonylethyl)amino-2,2-dimethylbutansäureesterderivat der allgemeinen Formel 5 isoliert
und

c) das so hergestellte 3-(2-Ethoxycarbonylethyl)amino-2,2-dimethylbutansäureesterderivat der allgemeinen Formel 5 in einem aliphatischen oder aromatischen Kohlenwasserstoff in Gegenwart eines Alkalialoholats eines geradkettigen oder verzweigten $C_1$-$C_4$-Alkohols den Bedingungen einer Dieckmann'schen Esterkondensation unterwirft, die flüchtigen Bestandteile der aus der Cyclisierungsreaktion resultierenden Reaktionsmischung destillativ entfernt, danach das Gemisch hydrolysiert und mit der wässerigen Lösung einer Mineralsäure versetzt, die resultierende Mischung mit einem mit Wasser nicht mischbaren Dialkylether und mit wässeriger Ammoniaklösung versetzt, die vereinigten organischen Extrakte einengt und das resultierende Piperidonderivat der allgemeinen Formel 6 isoliert

d) das Piperidonderivat 6 in einer Mischung aus einem geradkettigen oder verzweigten $C_1$-$C_4$-Alkohol und Wasser in Gegenwart eines Alkalihydroxyds oder einer Mineralsäure unter Erhitzen zum entsprechenden 3,3-Dimethylpiperidonderivat der allgemeinen Formel 7 verseift und decarboxyliert, das Produkt isoliert und gegebenenfalls mit einer Protonensäure das entsprechende Säureadditionssalz herstellt
und

e) das so erhaltene Stereoisomerengemisch gegebenenfalls nach Freisetzung der jeweiligen enantiomeren freien Basen in einem geradkettigen oder verzweigten $C_1$-$C_4$-Alkohol löst, mit dem entsprechenden Enantiomeren der Äpfelsäure,Weinsäure, Mandelsäure oder Campfersulfonsäure versetzt, das gewünschte Stereoisomere in Form seines Additionssalzes mit der optisch aktiven Säure isoliert, die das nicht gewünschte Isomere enthaltende Mutterlauge erhitzt und dabei das nicht gewünschte Enantiomere thermisch in das gewünschte Stereoisomere überführt, mit einer optisch aktiven, enantiomerenreinen, zur Bildung eines Säureadditionssalzes befähigten, organischen Säure versetzt und das so als Säureadditionssalz vorliegende gewünschte Stereoisomere gegebenenenfals durch Zugabe eines $C_3$-$C_8$-Alkohols kristallisiert und isoliert und diesen Vorgang gegebenenfalls wiederholt
und

f) das so erhaltene reine Stereoisomere nach dem Freisetzen aus dem enantiomerereinen Säureadditionssalz in einem - gegebenenfalls cyclischen - Ether mit einem Methyltriphenylphosphoniumhalogenid in Gegenwart eines Alkalialkoholats in einem Temperaturintervall von 20 bis 60 °C einer Wittig-Reaktion unterwirft, das Reaktionsgemisch nach erfolgter Umsetzung mit Wasser sowie mit einem Halogenalkan versetzt, die wässerige Phase erschöpfend extrahiert, das Reaktionsprodukt vom Typ 9 oder gegebenenfalls das entsprechende Stereoisomere nach Zugabe einer Protonensäure in Form seines Säureadditionssalzes isoliert
und

g) das aus der Wittig-Reaktion hervorgegangene Alken 9 gegebenenfalls zunächst aus seinem Säureadditionssalz freisetzt und die freie Base vom Typ 9 in einem Alkylaromaten als Lösungsmittel löst und mit einem Alkylformiat einer Formylierungsreaktion am Piperidinstickstoff unterwirft und das Reaktionsprodukt vom Typ 10 oder dessen entsprechendes Stereoisomere isoliert
und

h) die so gewonnene Formylverbindung 10 - bzw. das entsprechende Stereoisomere - in einem halogenierten Kohlenwasserstoff löst und mit einem Aluminium(III)halogenid umsetzt und das aus dieser Umsetzung resultierende Cyclisierungsprodukt vom Typ 11 isoliert
und

i) das aus der Cyclisierungsreaktion resultierende Benzomorphanderivat 11 in einem Alkanol löst und mit der wässerigen Lösung einer Halogenwasserstoffsäure zur Reaktion bringt und das aus dieser Umsetzung resultierende deformylierte Norbenzomorphan vom Typ 12 gegebenenfalls, nach Zugabe einer Protonensäure in Form seines Säureadditionssalzes isoliert
und

j) falls gewünscht, nach Freisetzung der freien Benzomorphan-Base den Substituenten $R_2$ - im Falle dieser eine Alkoxygruppe verkörpert - auf dem Wege einer Etherspaltung in eine freie Hydroxyfunktion überführt und das Reaktionsprodukt - gegebenenfalls in Form seines Säureadditionssalzes vom Typ 13, in dem die Base der allgemeinen Formel 1 entspricht, isoliert

**4.** Verfahren zur Herstellung von Norbenzomorphan der allgemeinen Formel 1, in der $R_1$ die in Anspruch 1 angegebene Bedeutung hat, nach Anspruch 3, dadurch gekennzeichnet, daß man

a) ein Benzylcyanid der allgemeinen Formel 2, in der $R_2$ die in Anspruch 1 angegebene Bedeutung hat - mit einem Bromisobutylbuttersäureester der allgemeinen Formel 3, in der $R_3$ $C_1$-$C_6$-Alkyl bedeutet, in Gegenwart von Chlortrimethylsilan und Zinkpulver in Dichlormethan nach dem Vedünnen mit Tetrahydrofuran umsetzt, die Reaktionsmischung erwärmt, nach erfolgter Reaktion abkühlen läßt, das Zinkpulver abtrennt und das Reaktionsgemisch mit Natriumcyanoborhydrid versetzt und das Reaktionsgemisch mit Ethanol verdünnt und danach mit konzentrierter wässeriger Ammoniaklösung versetzt, die organische Phase abtrennt und einengt, den Rückstand in Toluol aufnimmt, die erhaltene Lösung mit 2N Salzsäure extrahiert, die vereinigten wässerigen Extrakte mit konzentrierter wässeriger Ammoniaklösung alkalisch stellt, diese alkalische Lösung mit Dichlormethan extrahiert und das resultierende 3-Amino-2,2-dimethylbutansäureester-Derivat der allgemeinen Formel 4 isoliert
und

b) das 3-Amino-2,2-dimethylbutansäureesterderivat der allgemeinen Formel 4 mit einem Acrylsäureester, in dem die Alkoholkomponente $R_4$ die Bedeutung einer $C_1$-$C_6$-Alkylgruppe hat, einer Michael-Additionsreaktion in Ethanol als Lösungsmittel unterwirft, das Reaktionsmedium nach erfolgter Reaktion entfernt und das resultierende 3-(2-Ethoxycarbonylethyl)amino-2,2-dimethylbutansäureesterderivat der allgemeinen Formel 5 isoliert
und

c) das so hergestellte 3-(2-Ethoxycarbonylethyl)amino-2,2--dimethylbutansäureesterderivat der allgemeinen Formel 5 in Toluol in Gegenwart von Kalium-*tert*.-butanolat den Bedingungen einer Dieckmann'schen Esterkondensation unterwirft, die flüchtigen Bestandteile der aus der Cyclisierungsreaktion resultierenden Reaktionsmischung destillativ entfernt, danach das Gemisch hydrolysiert und mit konzentrierter Salzsäure versetzt, die resultierende Mischung mit Diethylether und mit konzentrierter Ammoniaklösung versetzt, die vereinigten organischen Extrakte einengt und das resultierende Piperidonderivat der allgemeinen Formel 6 isoliert
und

d) das Piperidonderivat 6 in einer Ethanol/Wasser-Mischung in Gegenwart von Natriumhydroxyd bzw. Salzsäure oder Schwefelsäure unter Erhitzen auf Rückflußtemperatur zum entsprechenden 3,3-Dimethylpiperidonderivat der allgemeinen Formel 7 verseift und decarboxyliert, das Reaktionsprodukt isoliert und gegebenenfalls mit Chlor- oder Bromwasserstoffsäure das entsprechende Hydrohalogenid herstellt
und

e) das so erhaltene Stereoisomerengemisch gegebenenfalls nach Freisetzung der jeweiligen enantiomeren

freien Basen in Ethanol löst, mit dem entsprechenden Enantiomeren (D- oder L-Form) der Weinsäure versetzt, das gewünschte Stereoisomere in Form des entsprechenden Tartrates isoliert, die das nicht gewünschte Isomere enthaltende Mutterlauge erhitzt und dabei das nicht gewünschte Enantiomere thermisch in das gewünschte Stereoisomere überführt, mit D- oder L-Weinsäure versetzt und das so als entsprechendes Tartrat vorliegende gewünschte Stereoisomere gegebenenenfals durch Zugabe von Isopropanol kristallisiert und den Niederschlag isoliert und diesen Vorgang gegebenenfalls wiederholt
und

f) das so erhaltene reine Stereoisomere nach dem Freisetzen aus dem enantiomerereinen Säureadditionssalz in Tetrahydrofuran mit Methyltriphenylphosphoniumbromid in Gegenwart von Kalium-*tert*.--butanolat bei einer Temperatur von 40 °C einer Wittig-Reaktion unterwirft, das Reaktionsgemisch nach erfolgter Umsetzung mit Wasser sowie mit Dichlormethan versetzt, die wässerige Phase erschöpfend extrahiert, das Reaktionsprodukt vom Typ 9 oder das entsprechende Stereoisomere in Form seines Hydrohalogenids isoliert
und

g) das aus der Wittig-Reaktion hervorgegangene Alken 9 gegebenenfalls zunächst aus seinem Säureadditionssalz freisetzt und die freie Base vom Typ 9 in Toluol löst und mit n-Butylformiat einer Formylierungsreaktion am Piperidinstickstoff unterwirft und das Reaktionsprodukt vom Typ 10 oder dessen entsprechendes Stereoisomere isoliert
und

h) die so gewonnene Formylverbindung 10 - bzw. das entsprechende Stereoisomere - in Dichlormethan löst und bei einer Temperatur von nicht größer als -5°C mit Aluminium(III)chlorid umsetzt und das aus dieser Umsetzung resultierende Cyclisierungsprodukt vom Typ 11 isoliert
und

i) das aus der Cyclisierungsreaktion resultierende Benzomorphanderivat 11 in n-Propanol löst und mit konzentrierter Salzsäure zur Reaktion bringt und das aus dieser Umsetzung resultierende deformylierte Norbenzomorphan vom Typ 12 in Form seines Hydrochlorids isoliert
und

j) falls gewünscht nach Freisetzung der freien Benzomorphan-Base den Substituenten $R_2$ - im Falle dieser eine Alkoxygruppe verkörpert - auf dem Wege einer Etherspaltung in eine freie Hydroxyfunktion überführt und das Reaktionsprodukt - gegebenenfalls nach Zugabe von Chlor- oder Bromwasserstoffsäure in Form seines Hydrohalogenids der allgemeinen Formel 13 , wobei die Base der allgemeinen Formel 1 entspricht, isoliert.

**5.** Verfahren zur Herstellung von Norbenzomorphan der allgemeinen Formel 1, in der $R_1$ eine Hxdroylgruppe bedeutet, die sich in 3'-Stellung befindet, nach Anspruch 4, dadurch gekennzeichnet, daß man

a) ein Benzylcyanid der allgemeinen Formel 2, in der $R_2$ die Bedeutung einer Methoxygruppe hat, die sich in 3-Stellung befindet, mit Bromisobutylbuttersäureethylester (3) ($R_3$=$C_2H_5$) in Gegenwart von Chlortrimethylsilan und Zinkpulver in Dichlormethan nach dem Verdünnen mit Tetrahydrofuran umsetzt, die Reaktionsmischung erwärmt, nach erfolgter Reaktion abkühlen läßt, das Zinkpulver abtrennt und das Reaktionsgemisch mit Natriumcyanoborhydrid versetzt und das Reaktionsgemisch mit Ethanol verdünnt und danach mit konzentrierter wässeriger Ammoniaklösung versetzt, die organische Phase abtrennt und einengt, den Rückstand in Toluol aufnimmt, die erhaltene Lösung mit 2N Salzsäure extrahiert, die vereinigten wässerigen Extrakte mit konzentrierter wässeriger Ammoniaklösung alkalisch stellt, diese alkalische Lösung mit Dichlormethan extrahiert und den resultierenden 3-Amino-4-(3-methoxyphenyl)-2,2-dimethylbutansäureethylester (4, $R_2$ = 3-$OCH_3$) isoliert
und

b) den 3-Amino-4-(3-methoxyphenyl)-2,2-dimethylbutansäureethylester (4, $R_2$= 3-$OCH_3$) mit Acrylsäureethylester (R4=$C_2H_5$) einer Michael-Additionsreaktion in Ethanol als Lösungsmittel unterwirft, das Reaktionsmedium nach erfolgter Reaktion entfernt und den 3-(2-Ethoxycarbonylethyl)amino-4-(3-methoxyphenyl)-2-dimethylbutansäureethylester (5, $R_2$=3-$OCH_3$) isoliert
und

c) den 3-(2-Ethoxycarbonylethyl)amino-4-(3-methoxyphenyl)-2-dimethylbutansäureethylester (5,

$R_2$=3-$OCH_3$) in Toluol in Gegenwart von Kalium-*tert*.-butanolat den Bedingungen einer Dieckmann'schen Esterkondensation unterwirft, die flüchtigen Bestandteile der aus der Cyclisierungsreaktion resultierenden Reaktionsmischung destillativ entfernt, danach das Gemisch hydrolysiert und mit konzentrierter Salzsäure versetzt, die resultierende Mischung mit Diethylether und mit konzentrierter Ammoniaklösung versetzt, die vereinigten organischen Extrakte einengt und das 5-Carboethoxy-3,3-dimethyl-2-(3-methoxyphenyl)methyl-4-piperidon (6, $R_2$=3-$OCH_3$) isoliert
und

d) das 5-Carboethoxy-3,3-dimethyl-2-(3-methoxyphenyl)methyl-4-piperidon (6, $R_2$=3-$OCH_3$) in einer Ethanol/Wasser-Mischung in Gegenwart von Natriumhydroxyd bzw. Salzsäure oder Schwefelsäure unter Erhitzen auf Rückflußtemperatur zum 2-(3-Methoxyphenyl)methyl-3,3-dimethyl-4-piperidon-hydrochlorid (7, $R_2$ = m-$CH_3O$) verseift und decarboxyliert, das Reaktionsprodukt isoliert und mit Chlorwasserstoffsäure das entsprechende 2-(3-Methoxyphenyl)methyl-3,3-dimethyl-4-piperidon-hydrochlorid fällt
und

e) das so erhaltene Stereoisomerengemisch des 2-(3-Methoxyphenyl)methyl-3,3-dimethyl-4-piperidon-hydrochlorids vom Typ 7 ($R_2$=3-$OCH_3$) nach Freisetzung der jeweiligen enantiomeren freien Basen in Ethanol löst, mit D-(-)-Weinsäure versetzt, das gewünschte (+)-2-(3-Methoxyphenyl)methyl-3,3-dimethyl-4-piperidonium-hydrogentartrat vom Typ 8A ($R_2$=3-$OCH_3$) isoliert, die das nicht gewünschte Isomere enthaltende Mutterlauge erhitzt und dabei das nicht gewünschte (-)-2-(3-Methoxyphenyl)methyl-3,3-dimethyl-4-piperidon (8B, $R_2$=3-$OCH_3$) thermisch in das gewünschte (+)-2-(3-Methoxyphenyl)methyl-3,3-dimethyl-4-piperidon (8A) überführt, mit D-(-)-Weinsäure versetzt und das gewünschte (+)-2-(3-Methoxyphenyl)methyl-3,3-dimethyl-4-piperidoniumhydrogentartrat vom Typ 8A ($R_2$=3-$OCH_3$) durch Zugabe von Isopropanol kristallisiert und den Niederschlag isoliert und diesen Vorgang wiederholt
und

f) das so erhaltene reine (+)-2-(3-Methoxyphenyl)methyl-3,3-dimethyl-4-piperidoniumhydrogentartrat vom Typ 8A ($R_2$=3-$OCH_3$) nach dem Freisetzen aus dem enantiomerereinen Säureadditionssalz in Tetrahydrofuran mit Methyltriphenylphosphoniumbromid in Gegenwart von Kalium-*tert*.-butanolat bei einer Temperatur von 40 °C einer Wittig-Reaktion unterwirft, das Reaktionsgemisch nach erfolgter Umsetzung mit Wasser sowie mit Dichlormethan versetzt, die wässerige Phase erschöpfend extrahiert, das (+)-2-(3-Methoxyphenyl)methyl-3,3-dimethyl-4-methylen-piperidin (9) isoliert und mit Chlorwasserstoffsäure in das (+)-2-(3-Methoxyphenyl) methyl-3,3-dimethyl-4-methylen-piperidinhydrochlorid vom Typ 9 ($R_2$=3-$OCH_3$) überführt
und

g) das aus der Wittig-Reaktion hervorgegangene (3-Methoxyphenyl)methyl-3,3-dimethyl-4-methylen-piperidin (9) ($R_2$=3-$OCH_3$) aus seinem Hydrochlorid freisetzt und die freie Base in Toluol löst und mit n-Butylformiat einer Formylierungsreaktion am Piperidinstickstoff unterwirft und das (+)-N-Formyl-2-(3-methoxyphenyl)methyl-3,3-dimethyl-4-methylen-piperidin vom Typ 10 ($R_2$=3-$OCH_3$) isoliert
und

h) das (+)-N-Formyl-2-(3-methoxyphenyl)methyl-3,3-dimethyl-4-methylen-piperidin vom Typ 10 ($R_2$=3-$OCH_3$) in Dichlormethan löst und mit Aluminium(III)chlorid umsetzt und das aus dieser Umsetzung resultierende (-)-2-Formyl-3'-methoxy-5,9,9-trimethyl-6,7-benzomorphan vom Typ 11 ($R_2$ = 3'-$CH_3O$) isoliert
und

i) das aus der Cyclisierungsreaktion resultierende (-)-2-Formyl-3'-methoxy-5,9,9-trimethyl-6,7-benzomorphan vom Typ 11 ($R_2$ = 3'-$CH_3O$) in n-Propanol löst und mit konzentrierter Salzsäure zur Reaktion bringt und das aus dieser Umsetzung resultierende (-)-3'-Methoxy-5,9,9-trimethyl-6,7-benzomorphan vom Typ 12 ($R_2$ = 3'-$CH_3O$) mit Chlorwasserstoffsäure in das Hydrochlorid überführt
und

j) nach Freisetzung des freien (-)-3'-Methoxy-5,9,9-trimethyl-6,7-benzomorphans vom Typ 12 ($R_2$ = 3'-$CH_3O$) aus dem Hydrochlorid die 3'-Methoxyfunktion mit wässeriger Bromwasserstoffsäure unter Rückflußbedingungen in eine freie Hydroxyfunktion überführt und das (-)-3'-Hydroxy-5,9,9-trimethyl-6,7-benzomorphan vom Typ 1 mit Bromwasserstoffsäure in das entsprechende (-)- -3'-Hydroxy-5,9,9-trimethyl-6,7-benzomorphan-hydrobromid der allgemeinen Formel 13, (R2=3'-OH), die der allgemeinen Formel 1 entspricht, überführt.

**6.** Verfahren nach einem der Ansprüche 1 bis 5, dadurch gekennzeichnet, daß im Falle $R_1$ die Bedeutung von Nitro, Cyano, $-NH_2$, $-NH(C_1-C_8\text{-Alkyl})$, $-N(C_1-C_8\text{-Alkyl})_2$, wobei die Alkylreste gleich oder verschieden sein können, -NH-Acyl-($C_1$-$C_8$-Alkyl), worin Acyl für Benzoyl oder einen Alkylcarbonylrest mit einem geradkettigen oder verzweigten Niederalkylrest mit 1 bis 6 Kohlenstoffatom(en), wobei der Alkylrest gegebenenfalls mit einem oder mehreren Halogenatom(en), die untereinander gleich oder verschieden sein können, substitiuert sein kann, hat,
eine Verbindung der allgemeinen Formel 12, in der $R_2$ Wasserstoff bedeutet, auf an sich bekannte Weise in die entsprechend funktionalisierte Verbindung der allgemeinen Formel 13 übergeführt wird.

## Claims

**1.** Process for preparing norbenzomorphans of general formula 1

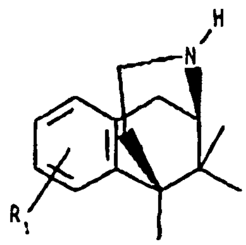

1

wherein

$R_1$ denotes hydrogen, $C_{1-6}$-alkyl, halogen, hydroxy, $C_{1-8}$-alkoxy, a benzoyl group bound via an oxygen or an alkylcarboxyl group having a straight-chained or branched $C_{1-6}$-lower alkyl group - wherein the alkyl group may optionally be substituted by one or more halogen atoms which may be identical or different -
nitro, cyano, $NH_2$, $NH(C_{1-8}\text{-alkyl})$, $N(C_{1-8}\text{-alkyl})_2$, wherein the alkyl groups may be identical or different, NH-acyl-($C_{1-8}$-alkyl), wherein acyl denotes benzoyl or an alkylcarbonyl group having a straight-chained or branched $C_{1-6}$-lower alkyl group, whilst the alkyl group may optionally be substituted by one or more halogen atoms which may be the same as one another or different from one another,

characterised in that

a) a benzylcyanide of general formula 2 wherein $R_2$ denotes hydrogen, $C_{1-6}$-alkyl, halogen, hydroxy, $C_{1-8}$-alkoxy, a benzoyl group bound via an oxygen or an alkylcarboxyl group having a straight-chained or branched lower alkyl group having 1 to 6 carbon atoms - where the alkyl group may optionally be substituted by one or more halogen atoms, which may be identical or different, - is subjected to the conditions of a Reformatsky reaction with ethyl bromoisobutylbutyrate of general formula 3 wherein $R_3$ denotes $C_{1-8}$-alkyl or benzyl, in the presence of an alkylhalosilane and zinc powder in an inert solvent in the presence of a reducing agent which is selective with regard to the reduction of imino functions, and the resulting ethyl 3-amino-2,2-dimethylbutanoate derivative of general formula 4 is isolated

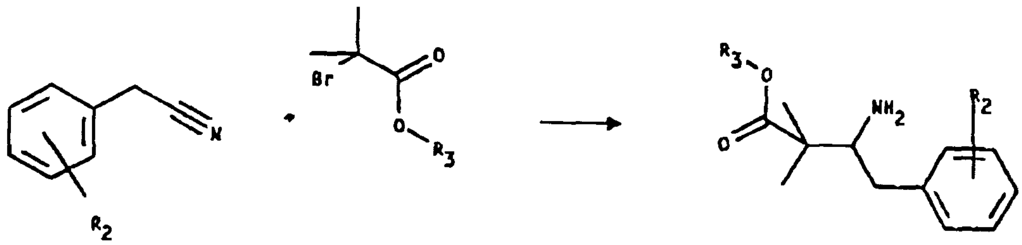

2 3 4

and

b) the 3-amino-2,2-dimethylbutanoic acid ester derivative of general formula 4 is subjected to the conditions of a Michael addition reaction with an acrylic acid ester wherein the alcohol component $R_4$ denotes a $C_{1-8}$-alkyl group or a benzyl group, and the resulting 3-(2-ethoxycarbonylethyl)amino-2,2-dimethylbutanoic acid ester derivative of general formula 5 is isolated

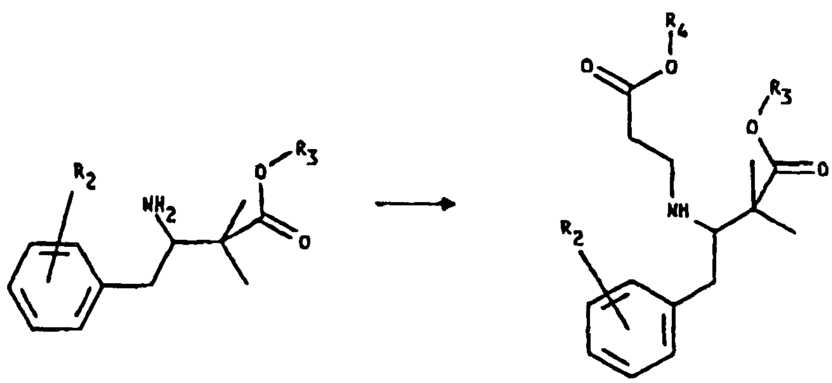

4 5

and

c) the 3-(2-ethoxycarbonylethyl)amino-2,2-dimethylbutanoic acid ester derivative of general formula 5 thus prepared is subjected to the conditions of a Dieckmann ester condensation in an inert solvent in the presence of a basic reacting compound and the resulting piperidone derivative of general formula 6 is isolated

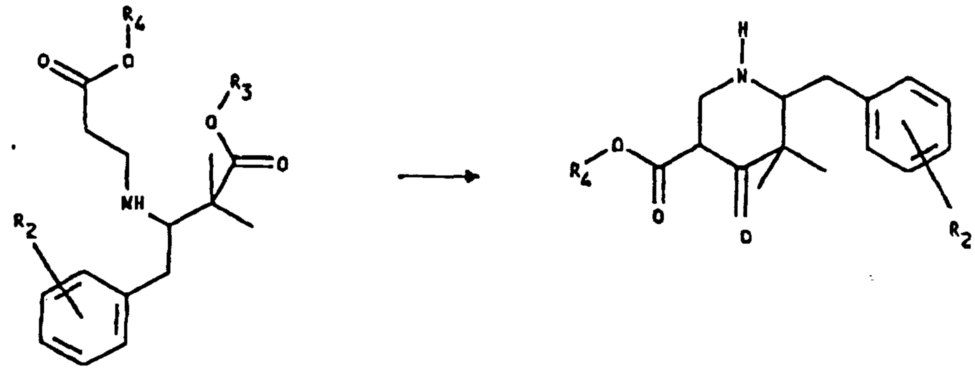

5 6

and

d) the piperidone derivative 6 is saponified under acid or alkaline conditions in a polar solvent or solvent mixture, then decarboxylated with heating, to obtain the corresponding 3,3-dimethylpiperidone derivative of general formula 7, isolated and if desired the corresponding acid addition salt is prepared using an acid and isolated

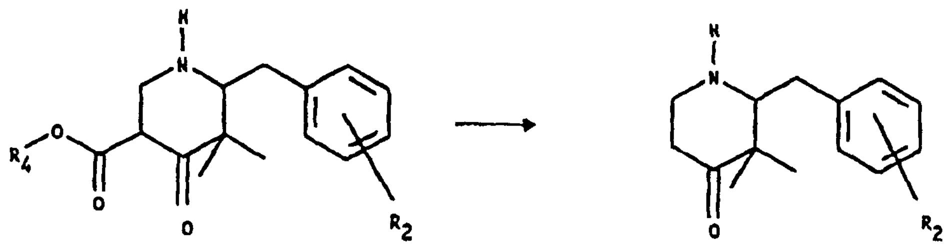

6 7

and

e) the stereoisomer mixture thus obtained is dissolved, optionally after release of the enantiomeric free bases, in a reaction medium which is inert with respect to enantiomer separation, then combined with a suitable stereoisomer of an organic acid suitable for salt formation with a stereoisomer of the mixture of enantiomers, the desired stereoisomer is isolated in the form of its addition salt with the optically active acid, the mother liquor containing the unwanted isomer is heated and in this way the unwanted enantiomer is converted thermally into the desired stereoisomer, mixed with an optically active enantiomerically pure organic acid capable of forming an acid addition salt and the desired stereoisomer thus present as an acid addition salt is optionally added, with the addition of a medium which behaves as a nonsolvent relative to the desired salt, and then isolated and this process is repeated as necessary

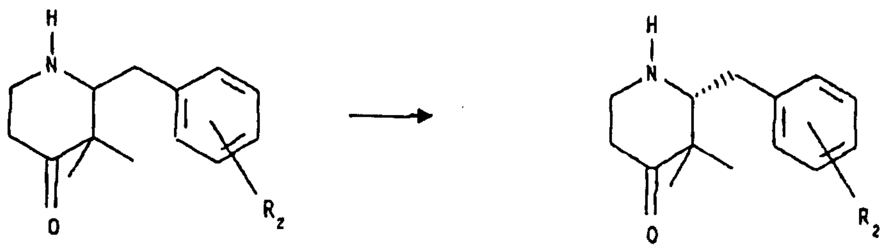

7

8A

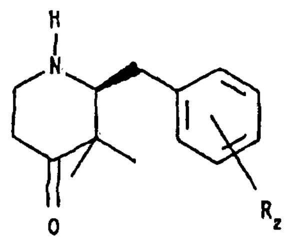

8B

and

f) the pure stereoisomer thus obtained, after release from the enantiomerically pure acid addition salt, is reacted in an inert solvent with a Wittig reagent generating a methylene group in the presence of a basically reacting compound and the reaction product of type 9 or the corresponding stereoisomer is isolated, optionally in the form of the acid addition salt thereof

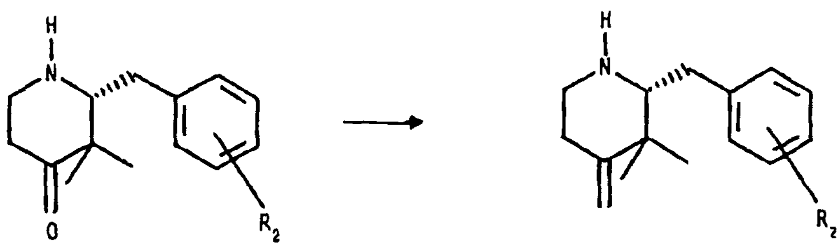

8

9

and

g) the alkene 9 obtained from the Wittig reaction is optionally first freed from its acid addition salt and the free base of type 9 is dissolved in an organic solvent and subjected, with a formylating agent, to a formylating reaction at the piperidine nitrogen and the reaction product of type 10 or the corresponding stereoisomer thereof is isolated

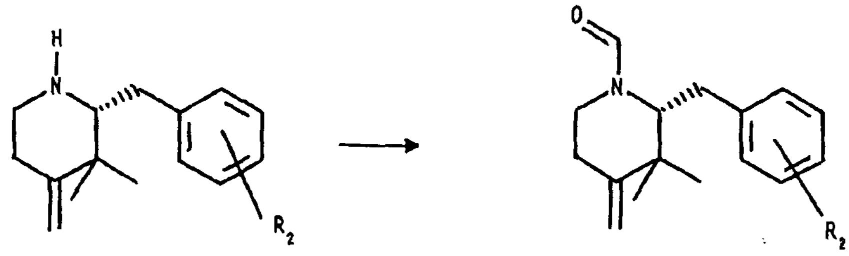

9 10

and

h) the formyl compound 10 thus obtained, or the corresponding stereoisomer, is dissolved in an inert solvent and reacted with a Lewis acid and the cyclising product of type 11 resulting from this reaction is isolated

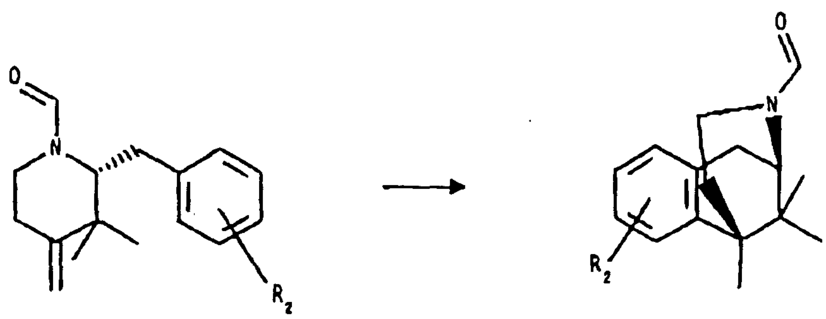

10 11

and

i) the benzomorphan derivative resulting from the cyclising reaction is dissolved in a polar solvent and reacted with an acidically reacting compound and the deformylated norbenzomorphan of type 12 resulting from this reaction is optionally isolated in the form of its acid addition salt

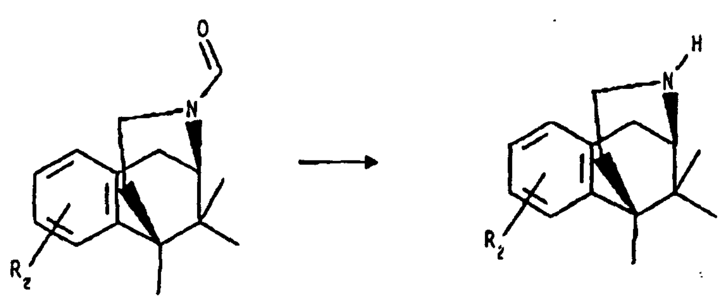

11 12

and

j) if desired, after liberation of the free benzomorphan base, the substituent $R_2$, if it constitutes an alkoxy group, is converted by ether splitting into a free hydroxy function and the reaction product is isolated, optionally in the form of the acid addition salt thereof of type 13, the base corresponding to general formula 1.

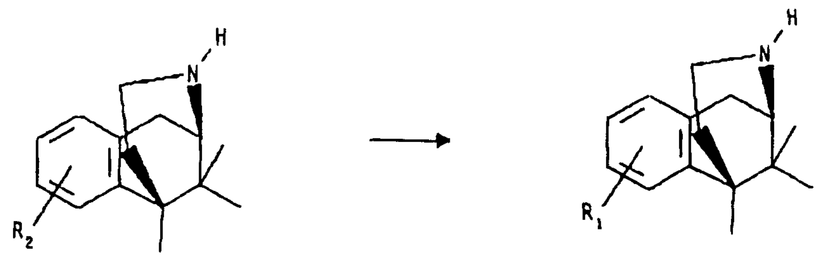

12 13

2. Process for preparing norbenzomorphan of general formula 1 according to claim 1 wherein $R_1$ has the meaning given in claim 1,
characterised in that

a) a benzylcyanide of general formula 2 wherein $R_2$ has the meaning given in claim 1 is reacted with a bromoisobutylbutyric acid ester of general formula 3 wherein $R_3$ denotes $C_{1-8}$-alkyl or benzyl, in the presence of an alkyl halosilane and zinc powder in an inert solvent, the reaction mixture is heated, allowed to cool after the reaction has ended, the zinc powder is separated off and the reaction mixture is mixed with a reducing agent which is selective with regard to the reduction of imino functions and the reaction mixture is diluted with an alkanol and then mixed with an aqueous solution of a basic reacting compound, the organic phase is removed and evaporated down, the residue is taken up in an inert solvent, the resulting solution is extracted with the aqueous solution of an acid, the combined aqueous extracts are made alkaline with a basic reacting compound, this alkaline solution is extracted with a water-immiscible organic solvent and the resulting ethyl 3-amino-2,2-dimethylbutanoic acid ester derivative of general formula 4 is isolated
and

b) the 3-amino-2,2-dimethylbutanoic acid ester derivative of general formula 4 is subjected with an acrylic acid ester wherein the alcohol component $R_4$ denotes a $C_{1-8}$-alkyl group or a benzyl group, to a Michael addition reaction in an inert solvent, the reaction medium is eliminated once the reaction has ended and the resulting ethyl 3-(2-ethoxycarbonylethyl)amino-2,2-dimethylbutanoate derivative of general formula 5 is isolated
and

c) the 3-(2-ethoxycarbonylethyl)amino-2,2-dimethylbutanoic acid ester derivative of general formula 5 thus prepared is subjected to the conditions of a Dieckmann ester condensation in an inert solvent in the presence of a basic reacting compound, the volatile components of the reaction mixture resulting from the reaction of cyclisation are eliminated by distillation, then the mixture is hydrolysed and mixed with the aqueous solution of an acidically reacting compound, the resulting mixture is combined with a water-immiscible organic solvent and an aqueous solution of a basically reacting compound, the combined organic extracts are evaporated down and the resulting piperidone derivative of general formula 6 is isolated
and

d) the piperidone derivative 6 is saponified under acid or alkaline conditions in a polar solvent or mixture of solvents, then decarboxylated with heating, to obtain the corresponding 3,3-dimethylpiperidone derivative of general formula 7 and isolated and if desired the corresponding acid addition salt is prepared using an acid and then isolated
and

e) the mixture of stereoisomers thus obtained is dissolved, optionally after liberation of the enantiomeric free bases, in a reaction medium which is inert relative to the enantiomer separation, mixed with a suitable stereoisomer of an organic acid which is suitable for salt formation with a stereoisomer of the enantiomer mixture, the desired stereoisomer is isolated in the form of the acid addition salt thereof with the optically active acid, the mother liquor containing the unwanted isomer is heated and the unwanted enantiomer is thereby converted thermally into the desired stereoisomer, mixed with an optically active, enantiomerically pure, organic acid capable of forming an acid addition salt and the desired stereoisomer, thus present as an acid addition salt, is optionally added, with the addition of a medium which behaves as a nonsolvent relative to the desired salt,

and the salt is isolated and this process is repeated as necessary
and

f) the pure stereoisomer thus obtained, after liberation from the enantiomerically pure acid addition salt, is subjected to a Wittig reaction in an inert solvent with a Wittig reagent generating a methylene group in the presence of a basic reacting compound and in a temperature range from 0 to 80°C, after the reaction the mixture is combined with water and a water-immiscible organic solvent, the aqueous phase is extracted exhaustively, the reaction product of type 9 is isolated or, after the addition of a proton acid, the corresponding stereoisomer is isolated in the form of the acid addition salt thereof
and

g) the alkene 9 obtained from the Wittig reaction is optionally first freed from its acid addition salt and the free base of type 9 is dissolved in an organic solvent and subjected, with a formylating agent, to a formylating reaction at the piperidine nitrogen and the reaction product of type 10 or the corresponding stereoisomer thereof is isolated
and

h) the formyl compound 10 thus obtained, or the corresponding stereoisomer, is dissolved in an inert solvent and reacted with a Lewis acid and the cyclising product of type 11 resulting from this reaction is isolated
and

i) the benzomorphan derivative resulting from the cyclising reaction is dissolved in a polar solvent and reacted with an acidically reacting compound and the deformylated norbenzomorphan of type 12 resulting from this reaction is optionally isolated in the form of its acid addition salt, after the addition of the inorganic acid
and

j) if desired, after liberation of the free benzomorphan base, the substituent $R_2$, if it constitutes an alkoxy group, is converted by ether splitting into a free hydroxy function and the reaction product is isolated, optionally in the form of the acid addition salt thereof of type 13, the base corresponding to general formula 1.

**3.** Process for preparing norbenzomorphan of general formula 1, wherein $R_1$ is defined as in claim 1, according to claim 2, characterised in that

a) a benzylcyanide of general formula 2 wherein $R_2$ has the meaning given in claim 1 is reacted with a bromoisobutylbutyric acid ester of general formula 3 wherein $R_3$ denotes $C_{1-6}$-alkyl, in the presence of a trialkylhalosilane and zinc power in an ether and a haloalkane, the reaction mixture is heated, allowed to cool once the reaction has ended, the zinc powder is removed and the reaction mixture is combined with a complex alkali metal borohydride derivative which is selective with regard to the reduction of imino functions, and the reaction mixture is diluted with a $C_{1-4}$-alcohol and then with an aqueous ammonia solution, the organic phase is separated and evaporated down, the residue is taken up in an aliphatic or aromatic hydrocarbon, the solution obtained is extracted with the aqueous solution of an inorganic acid, the combined aqueous extracts are made alkaline with aqueous ammonia solution, this alkaline solution is extracted with a halohydrocarbon and the resulting ether 3-amino-2,2-dimethylbutanoate derivative of general formula 4 is isolated
and

b) the ethyl 3-amino-2,2-dimethylbutanoate derivative of general formula 4 is subjected to a Michael addition reaction with an acrylic acid ester, wherein the alcohol component $R_4$ denotes a $C_{1-8}$ alkyl group or a benzyl group, in a straight-chained or branched $C_{1-4}$-alcohol, after the reaction has ended the reaction medium is eliminated and the resulting 3-(2-ethoxycarbonylethyl)amino-2,2-dimethylbutanoic acid ester derivative of general formula 5 is isolated
and

c) the ethyl 3-(2-ethoxycarbonylethyl)amino-2,2-dimethylbutanoate derivative of general formula 5 thus prepared is subjected to the conditions of a Dieckmann ester condensation in an aliphatic or aromatic hydrocarbon in the presence of an alkali metal alkoxide of a straight-chained or branched $C_{1-4}$-alcohol, the volatile components of the reaction mixture resulting from the reaction of cyclisation are eliminated by distillation, then the mixture is hydrolysed and mixed with the aqueous solution of an inorganic acid, the resulting mixture is combined with a waterimmiscible dialkylether and with aqueous ammonia solution, the combined organic extracts

are evaporated down and the resulting piperidone derivative of general formula 6 is isolated
and

d) the piperidone derivative 6 is saponified in a mixture of a straight-chained or branched $C_{1-4}$-alcohol and water in the presence of an alkali metal hydroxide or an inorganic acid, and decarboxylated with heating, to obtain the corresponding 3,3-dimethylpiperidone derivative of general formula 7, the product is isolated and optionally the corresponding acid addition salt is prepared with a proton acid
and

e) the mixture of stereoisomers thus obtained is dissolved, optionally after liberation of the enantiomeric free bases, in a straight-chained or branched $C_{1-4}$-alcohol, mixed with the corresponding enantiomers of malic, tartaric, mandelic or camphor sulphonic acid, the desired stereoisomer is isolated in the form of its salt of addition with the optically active acid, the mother liquor containing the unwanted isomer is heated and in this way the unwanted enantiomer is thermally converted into the desired stereoisomer, mixed with an optically active, enantiomerically pure organic acid capable of forming an acid addition salt and the desired stereoisomer thus occurring as an acid addition salt is optionally crystallised by the addition of a $C_{3-8}$-alcohol and then isolated and this procedure is repeated as necessary
and

f) the pure stereoisomer thus obtained is liberated from the enantiomerically pure acid addition salt and then subjected to a Wittig reaction, in an optionally cyclic ether with a methyl triphenylphosphonium halide in the presence of an alkali metal alkoxide in a temperature range from 20 to 60°C, then after the reaction has taken place the reaction mixture is mixed with water and with a haloalkane, the aqueous phase is extracted exhaustively, the reaction product of type 9 or optionally the corresponding stereoisomer is isolated in the form of its acid addition salt after the addition of a proton acid
and

g) the alkene 9 obtained from the Wittig reaction is optionally first liberated from its acid addition salt and the free base of type 9 is dissolved in an alkyl aromatic compound as solvent and subjected, with an alkyl formate, to a formylation reaction at the piperidine nitrogen and the reaction product of type 10 or the corresponding stereoisomer thereof is isolated
and

h) the formyl compound 10 thus obtained, or the corresponding stereoisomer, is dissolved in a halogenated hydrocarbon and reacted with an aluminium(III)halide and the cyclisation product of type 11 resulting from this reaction is isolated
and

i) the benzomorphan derivative 11 resulting from the reaction of cyclisation is dissolved in an alkanol and reacted with the aqueous solution of a hydrohalic acid and the deformylated norbenzomorphan of type 12 resulting from this reaction is isolated optionally after the addition of a proton acid, in the form of the acid addition salt thereof
and

j) if desired, after liberation of the free benzomorphan base, the substituent $R_2$, if it denotes an alkoxy group, is converted into a free hydroxy function by the method of ether splitting and the reaction product is isolated, optionally in the form of the acid addition salt thereof of type 13, in which the base corresponds to general formula 1.

**4.** Process for preparing norbenzomorphan of general formula 1 wherein $R_1$ is defined as in claim 1, according to claim 3, characterised in that

a) a benzylcyanide of general formula 2 wherein $R_2$ has the meaning given in claim 1 is reacted with a bromoisobutylbutyric acid ester of general formula 3 wherein $R_3$ denotes $C_{1-6}$-alkyl, in the presence of chlorotrimethylsilane and zinc powder in dichloromethane after diluting with tetrahydrofuran, the reaction mixture is heated, then when the reaction has ended it is allowed to cool, the zinc powder is separated off and the reaction mixture is combined with sodium cyanoborohydride and the resulting mixture is diluted with ethanol and then mixed with concentrated aqueous ammonia solution, the organic phase is separated off and evaporated down,

the residue is taken up in toluene, the solution obtained is extracted with 2N hydrochloric acid, the combined aqueous extracts are made alkaline with concentrated aqueous ammonia solution, this alkaline solution is extracted with dichloromethane and the resulting 3-amino-2,2-dimethylbutanoic acid ester derivative of general formula 4 is isolated
and

b) the 3-amino-2,2-dimethylbutanoic acid ester derivative of general formula 4 is subjected to a Michael addition reaction with an acrylic acid ester, wherein the alcohol component $R_4$ denotes a $C_{1-6}$-alkyl group, in ethanol as solvent, the reaction medium is removed once the reaction has ended and the resulting 3-(2-ethoxycarbonylethyl)amino-2,2-dimethylbutanoic acid ester derivative of general formula 5 is isolated
and

c) the 3-(2-ethoxycarbonylethyl)amino-2,2-dimethylbutanoic acid ester derivative of general formula 5 thus obtained is subjected to the conditions of a Dieckmann ester condensation in toluene in the presence of potassium tert.-butoxide, the volatile components of the reaction mixture resulting from the reaction of cyclisation are eliminated by distillation, then the mixture is hydrolysed and combined with concentrated hydrochloric acid, the resulting mixture is combined with diethylether and concentrated ammonia solution, the combined organic extracts are evaporated down and the resulting piperidone derivative of general formula 6 is isolated
and

d) the piperidone derivative 6 is saponified in an ethanol/water mixture in the presence of sodium hydroxide or hydrochloric acid or sulphuric acid and decarboxylated whilst heating to reflux temperature, to obtain the corresponding 3,3-dimethylpiperidone derivative of general formula 7, the reaction product is isolated and the corresponding hydrohalide is optionally prepared with hydrochloric or hydrobromic acid
and

e) the mixture of stereoisomers thus obtained is dissolved in ethanol, optionally after liberation of the enantiomeric free bases, mixed with the corresponding enantiomer (D- or L-form) of tartaric acid, the desired stereoisomer is isolated in the form of the corresponding tartrate, the mother liquor containing the unwanted isomer is heated and in this way the unwanted enantiomer is converted thermally into the desired stereoisomer, then mixed with D- or L-tartaric acid and the desired stereoisomer thus present as the corresponding tartrate is optionally crystallised by the addition of isopropanol and the precipitate is isolated and this procedure is repeated as necessary
and

f) the pure stereoisomer thus obtained, after liberation from the enantiomerically pure acid addition salt, is subjected to a Wittig reaction in tetrahydrofuran with methyl triphenylphosphonium bromide in the presence of potassium tert.-butoxide at a temperature of 40°C, then when the reaction has ended the mixture is combined with water and dichloromethane, the aqueous phase is extracted exhaustively, the reaction product of type 9 or the corresponding stereoisomer is isolated in the form of its hydrohalide
and

g) the alkene 9 obtained from the Wittig reaction is optionally first liberated from its acid addition salt and the free base of type 9 is dissolved in toluene and subjected with n-butylformate to a reaction of formylation at the piperidine nitrogen and the reaction product of type 10 or the corresponding stereoisomer thereof is isolated
and

h) the formyl compound 10 - or the corresponding stereoisomer - thus obtained is dissolved in dichloromethane and reacted with aluminium(III)chloride at a temperature of not more than -5°C and the cyclisation product of type 11 resulting from this reaction is isolated
and

i) the benzomorphan derivative 11 resulting from the reaction of cyclisation is dissolved in n-propanol and reacted with concentrated hydrochloric acid and the deformylated norbenzomorphan of type 12 resulting from this reaction is isolated in the form of its hydrochloride
and

j) if desired, after liberation of the free benzomorphan base, the substituent $R_2$, if it denotes an alkoxy group,

is converted by ether splitting into a free hydroxy function and the reaction product is isolated, optionally after the addition of hydrochloric or hydrobromic acid, in the form of its hydrohalide of general formula 13, the base corresponding to general formula 1.

**5.** Process for preparing norbenzomorphan of general formula 1 wherein $R_1$ denotes a hydroxy group in the 3'-position, according to claim 4, characterised in that

a) a benzylcyanide of general formula 2 wherein $R_2$ denotes a methoxy group in the 3-position is reacted with ethyl bromoisobutylbutyrate (3) ($R_3 = C_2H_5$) in the presence of chlorotrimethylsilane and zinc powder in dichloromethane after dilution with tetrahydrofuran, the reaction mixture is heated, left to cool after the reaction has ended, the zinc powder is separated off and the reaction mixture is combined with sodium cyanoborohydride and then diluted with ethanol and then mixed with concentrated aqueous ammonia solution, the organic phase is separated off and evaporated down, the residue is taken up in toluene, the solution obtained is extracted with 2N hydrochloric acid, the combined aqueous extracts are made alkaline with concentrated aqueous ammonia solution, this alkaline solution is extracted with dichloromethane and the resulting ethyl 3-amino-4-(3-methoxyphenyl)-2,2-dimethylbutanoate (4, $R_2$ = 3-$OCH_3$) is isolated
and

b) the ethyl 3-amino-4-(3-methoxyphenyl)-2,2-dimethylbutanoate (4, $R_2$ = 3-$OCH_3$) is subjected with ethyl acrylate ($R_4 = C_2H_5$) to a Michael addition reaction in ethanol as solvent, then when the reaction has ended the reaction medium is eliminated and the ethyl 3-(2-ethoxycarbonylethyl)amino-4-(3-methoxyphenyl)-2-dimethylbutanoate (5, $R_2$ = 3-$OCH_3$) is isolated
and

c) the ethyl 3-(2-ethoxycarbonylethyl)amino-4-(3-methoxyphenyl)-2-dimethylbutanoate (5, $R_2$ = 3-$OCH_3$) is subjected to the conditions of a Dieckmann ester condensation in toluene in the presence of potassium tert.-butoxide, the volatile components of the reaction mixture resulting from the reaction of cyclisation are removed by distillation, after which the mixture is hydrolysed and mixed with concentrated hydrochloric acid, the resulting mixture is combined with diethylether and with concentrated ammonia solution, the combined organic extracts are evaporated down and the 5-carboethoxy-3,3-dimethyl-2-(3-methoxyphenyl)methyl-4-piperidone (6, $R_2$ = 3-$OCH_3$) is isolated
and

d) the 5-carboethoxy-3,3-dimethyl-2-(3-methoxyphenyl)methyl-4-piperidone (6, $R_2$ = 3-$OCH_3$) is saponified in an ethanol/water mixture in the presence of sodium hydroxide or hydrochloric acid or sulphuric acid and decarboxylated with heating to reflux temperature, to obtain the 2-(3-methoxyphenyl)methyl-3,3-dimethyl-4-piperidone-hydrochloride (7, $R_2$ = m-$CH_3O$), the reaction product is isolated and the corresponding 2-(3-methoxyphenyl)methyl-3,3-dimethyl-4-piperidone-hydrochloride is precipitated with hydrochloric acid
and

e) the stereoisomer mixture of the 2-(3-methoxyphenyl)methyl-3,3-dimethyl-4-piperidone-hydrochloride of type 7 ($R_2$ = 3-$OCH_3$) thus obtained is dissolved in ethanol, after the enantiomeric free bases have been liberated, then mixed with D-(-)-tartaric acid, the desired (+)-2-(3-methoxyphenyl)methyl-3,3-dimethyl-4-piperidonium hydrogen tartrate of type 8A ($R_2$ = 3-$OCH_3$) is isolated, the mother liquor containing the unwanted isomer is heated and in this way the unwanted (-)-2-(3-methoxyphenyl)methyl-3,3-dimethyl-4-piperidone (8B, $R_2$ = 3-$OCH_3$) is thermally converted into the desired (+)-2-(3-methoxyphenyl)methyl-3,3-dimethyl-4-piperidone (8A), mixed with D-(-)-tartaric acid and the desired (+)-2-(3-methoxyphenyl)methyl-3,3-dimethyl-4-piperidonium hydrogen tartrate of type 8A ($R_2$ = 3-$OCH_3$) is crystallised by the addition of isopropanol and the precipitate is isolated and this procedure is repeated
and

f) the pure (+)-2-(3-methoxyphenyl)methyl-3,3-dimethyl-4-piperidonium hydrogen tartrate of type 8A ($R_2$ = 3-$OCH_3$) thus obtained is liberated from the enantiomerically pure acid addition salt and then subjected to a Wittig reaction in tetrahydrofuran with methyl triphenylphosphonium bromide in the presence of potassium tert.-butoxide at a temperature of 40°C, after the reaction is complete the mixture is combined with water and with dichloromethane, the aqueous phase is extracted exhaustively, the (+)-2-(3-methoxyphenyl)methyl-3,3-dimethyl-4-methylene-piperidine (9) is isolated and converted with hydrochloric acid into the (+)-2-(3-methoxyphenyl)methyl-3,3-dimethyl-4-methylene-piperidine hydrochloride of type 9 ($R_2$ = 3-$OCH_3$)

and

g) the (3-methoxyphenyl)methyl-3,3-dimethyl-4-methylene-piperidine (9) ($R_2$ = 3-$OCH_3$) obtained from the Wittig reaction is liberated from its hydrochloride and the free base is dissolved in toluene and subjected with n-butylformate to a reaction of formylation at the piperidine nitrogen and the (+)-N-formyl-2-(3-methoxyphenyl) methyl-3,3-dimethyl-4-methylene-piperidine of type 10 ($R_2$ = 3-$OCH_3$) is isolated
and

h) the (+)-N-formyl-2-(3-methoxyphenyl)methyl-3,3-dimethyl-4-methylene-piperidine of type 10 ($R_2$ = 3-$OCH_3$) is dissolved in dichloromethane and reacted with aluminium(III)chloride and the (-)-2-formyl-3'-methoxy-5,9,9-trimethyl-6,7-benzomorphan of type 11 ($R_z$ = 3'-$CH_3O$) resulting from this reaction is isolated
and

i) the (-)-2-formyl-3'-methoxy-5,9,9-trimethyl-6,7-benzomorphan of type 11 ($R_2$ = 3'-$CH_3O$) resulting from the cyclisation is dissolved in n-propanol and reacted with concentrated hydrochloric acid and the (-)-3'-methoxy-5,9,9-trimethyl-6,7-benzomorphan of type 12 ($R_2$ = 3'-$CH_3O$) resulting from this reaction is converted with hydrochloric acid into the hydrochloride
and

j) after liberation of the free (-)-3'-methoxy-5,9,9-trimethyl-6,7-benzomorphan of type 12 ($R_2$ = 3'-$CH_3O$) from the hydrochloride, the 3'-methoxy function is converted with aqueous hydrobromic acid into a free hydroxy function under reflux conditions and the (-)-3'-hydroxy-5,9,9-trimethyl-6,7-benzomorphan of type 1 is converted with hydrobromic acid into the corresponding (-)-3'-hydroxy-5,9,9-trimethyl-6,7-benzomorphan-hydrobromide of general formula 13 ($R_2$ = 3'-OH) which corresponds to general formula 1.

**6.** Process according to one of claims 1 to 5, characterised in that, if $R_1$ denotes nitro, cyano, -$NH_2$, -NH($C_{1-8}$-alkyl), -N($C_{1-8}$-alkyl)$_2$ wherein the alkyl radicals may be the same or different, -NH-acyl-($C_{1-8}$-alkyl), wherein acyl may also denote a benzoyl radical or an alkyl-carbonyl radical having a branched or unbranched lower alkyl radical having 1 to 6 carbon atom(s), wherein the alkyl radical may be substituted by one or more halogen atom(s) which may be the same or differet,
a compound of general formula 12 wherein $R_2$ denotes hydrogen is processed by methods known per se to give a compound of general formula 13 which is functionalised correspondingly.

## Revendications

**1.** Procédé de préparation de norbenzomorphanes de formule générale 1

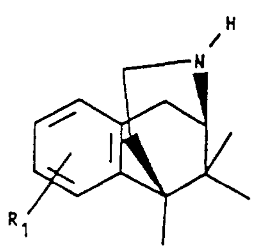

1

où

$R_1$ peut représenter l'hydrogène, alkyle en $C_1$-$C_6$, halogène, hydroxyle, alcoxy en $C_1$-$C_8$, un reste benzoyle lié par un oxygène ou un reste alkylcarboxyle avec un reste alkyle inférieur linéaire ou ramifié de 1 à 6 atomes de carbone, où le reste alkyle peut éventuellement être substitué par un ou plusieurs atomes d'halogène qui peuvent être identiques ou différents,
nitro, cyano, -$NH_2$, -NH(alkyle en $C_1$-$C_8$), -NH(alkyle en $C_1$-$C_8$)$_2$, où les restes alkyle peuvent être identiques ou différents, -NH-acyl-(alkyle en $C_1$-$C_8$), où acyle peut représenter benzoyle ou un reste alkylcarbonyle avec

un reste alkyle inférieur linéaire ou ramifié de 1 à 6 atomes de carbone, où le reste alkyle peut éventuellement être substitué par un ou plusieurs atomes d'halogène qui peuvent être identiques ou différents,

caractérisé en ce que

a) on soumet un cyanure de benzyle de formule générale 2 dans laquelle $R_2$ peut représenter l'hydrogène, alkyle en $C_1$-$C_6$, halogène, hydroxyle, alcoxy en $C_1$-$C_8$, un reste benzoyle lié par un oxygène ou un reste alkylcarboxyle avec un reste alkyle inférieur linéaire ou ramifié de 1 à 6 atomes de carbone, où le reste alkyle peut éventuellement être substitué par un ou plusieurs atomes d'halogène qui peuvent être identiques ou différents, avec un ester d'acide bromoisobutyrique de formule générale 3 dans laquelle $R_3$ représente alkyle en $C_1$-$C_8$ ou benzyle, en présence d'un alkylhalogénosilane et de poudre de zinc dans un solvant inerte et en présence d'un réducteur sélectif à l'égard de la réduction des fonctions imino aux conditions d'une réaction de Reformatsky et on isole le dérivé d'éthylester d'acide 3-amino-2,2-diméthylbutanoïque résultant de formule générale 4

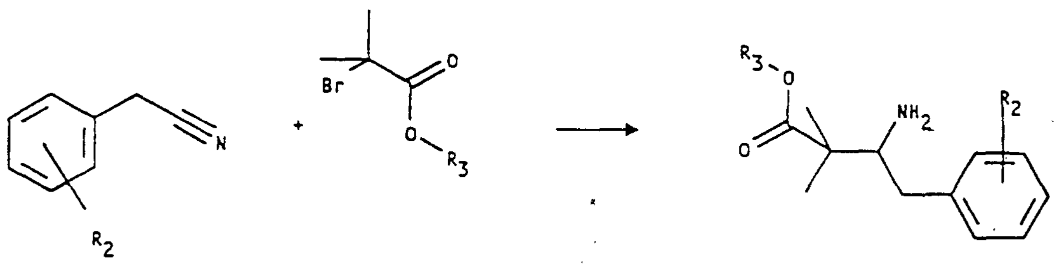

2 3 4

et

b) on soumet le dérivé d'ester d'acide 3-amino-2,2-diméthylbutanoïque de formule générale 4 avec un ester d'acide acrylique dans lequel le composant alcool $R_4$ a la signification d'un groupe alkyle en $C_1$-$C_8$ ou d'un groupe benzyle aux conditions d'une réaction d'addition de Michael et on isole le dérivé d'ester d'acide 3-(2-éthoxycarbonyléthyl)amino-2,2-diméthyl-butanoïque de formule générale 5

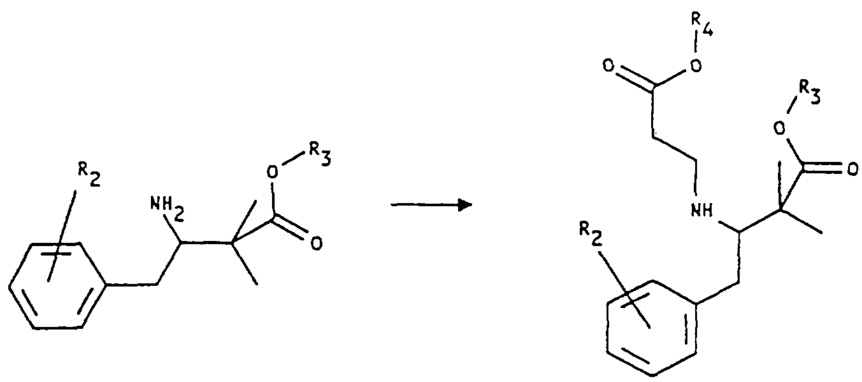

4 5

et

c) on soumet le dérivé d'ester d'acide 3-(2-éthoxycarbonyléthyl)amino-2,2-diméthylbutanoïque de formule générale 5 ainsi préparé dans un solvant inerte en présence d'un composé à réaction basique aux conditions d'une condensation d'ester de Dieckmann et on isole le dérivé de pipéridone résultant de formule générale 6

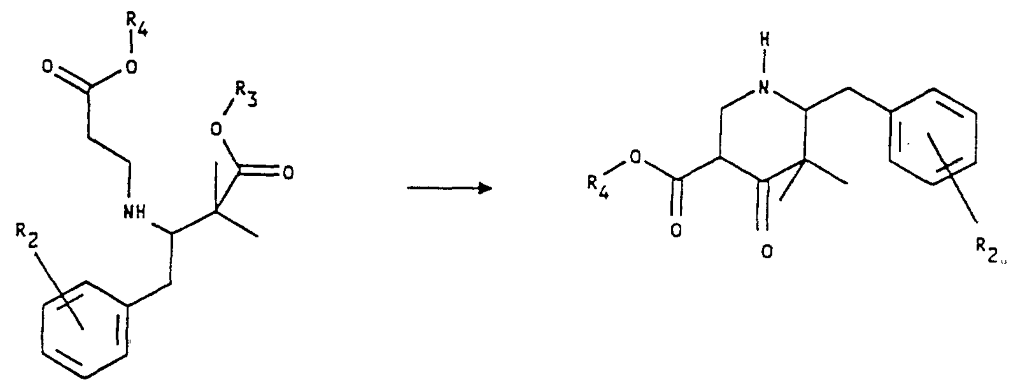

5 6

et

d) on saponifie et décarboxyle le dérivé de pipéridone 6 dans des conditions acides ou alcalines dans un solvant ou mélange de solvants polaire avec chauffage en le dérivé de 3,3-diméthylpipéridone correspondant de formule générale 7, on l'isole et, éventuellement, avec un acide on prépare le sel d'addition d'acide correspondant et on l'isole

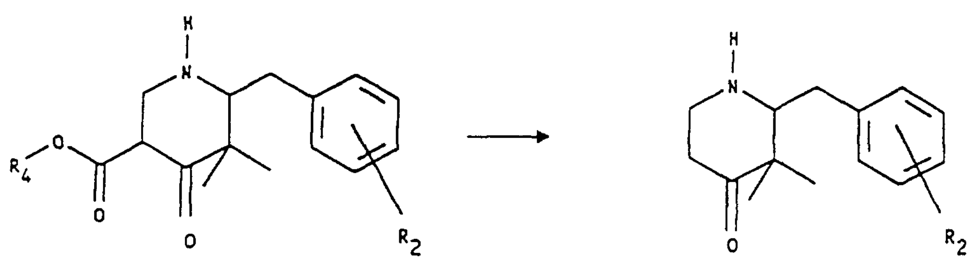

6 7

et

e) on dissout le mélange de stéréoisomères ainsi obtenu, éventuellement après libération des bases libres énantiomères correspondantes, dans un milieu réactionnel inerte à l'égard de la séparation d'énantiomères, on ajoute un stéréoisomère approprié d'un acide organique approprié à la salification avec un stéréoisomère du mélange d'énantiomères, on isole le stéréoisomère souhaité sous forme de son sel d'addition avec l'acide optiquement actif, on chauffe la liqueur mère contenant l'isomère non souhaité et on convertit alors thermiquement l'énantiomère non souhaité en le stéréoisomère souhaité, on ajoute un acide organique optiquement actif, énantiomériquement pur, capable de former un sel d'addition d'acide et on ajoute et isole le stéréoisomère souhaité ainsi présent sous forme de sel d'addition d'acide éventuellement avec addition d'un milieu qui se comporte comme un non-solvant à l'égard du sel souhaité, et on répète éventuellement ce processus

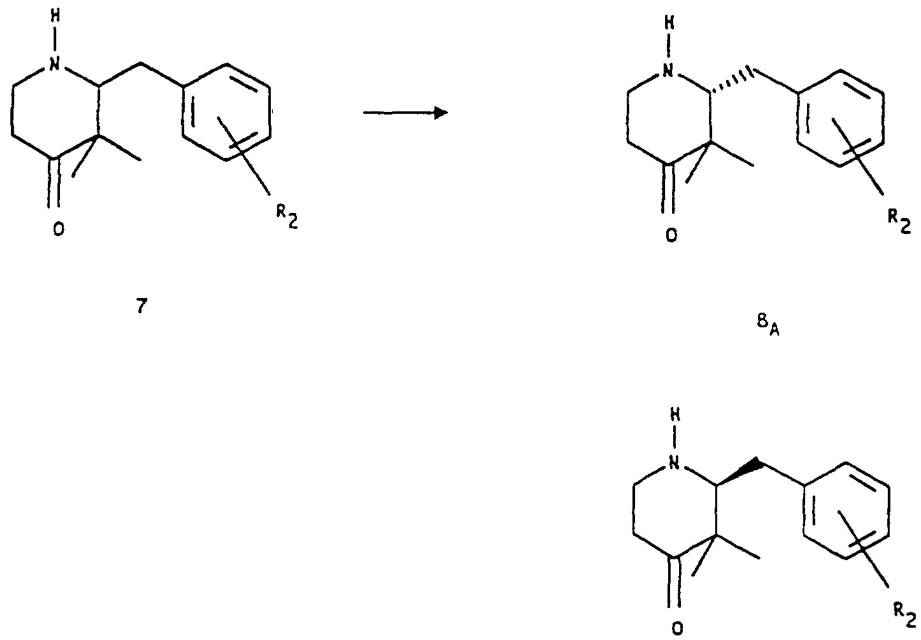

7

$8_A$

$8_B$

et

f) après la libération à partir du sel d'addition d'acide énantiomériquement pur, on soumet le stéréoisomère pur ainsi obtenu dans un solvant inerte avec un réactif de Wittig générant un groupe méthylène en présence d'un composé à réaction basique et on isole le produit réactionnel de type 9 ou le stéréoisomère correspondant éventuellement sous forme de son sel d'addition d'acide

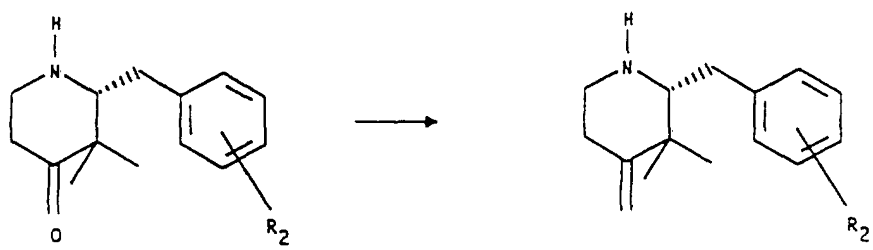

8 9

et

g) éventuellement on libère d'abord à partir de son sel d'addition d'acide l'alcène 9 issu de la réaction de Wittig et on dissout la base libre de type 9 dans un solvant organique et on la soumet avec un agent de formylation à une réaction de formylation sur l'azote de pipéridine et on isole le produit réactionnel de type 10 ou son stéréoisomère correspondant

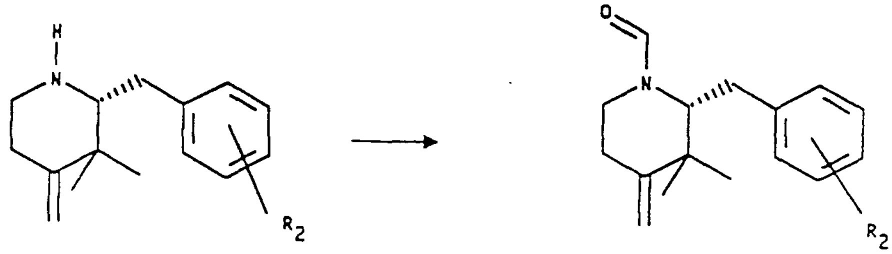

9 10

et

h) on dissout le composé formylé 10 ainsi obtenu, ou le stéréoisomère correspondant, dans un solvant inerte et on le fait réagir avec un acide de Lewis et on isole le produit de cyclisation de type 11 résultant de cette réaction

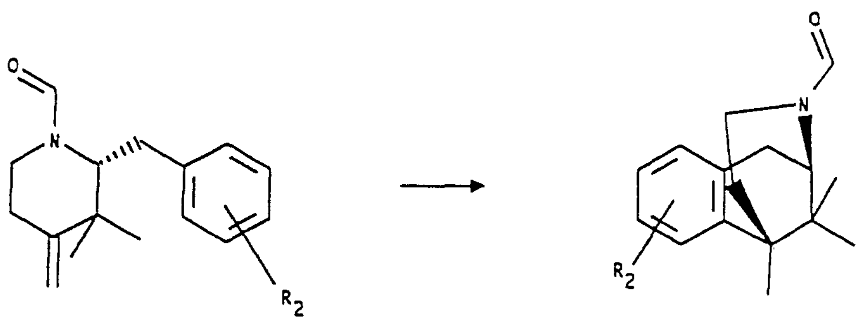

10 11

et

i) on dissout dans un solvant polaire de dérivé de benzomorphane résultant de la réaction de cyclisation et on le fait réagir avec un composé à réaction acide et on isole le norbenzomorphane de type 12 déformylé résultant de cette réaction éventuellement sous forme de son sel d'addition d'acide

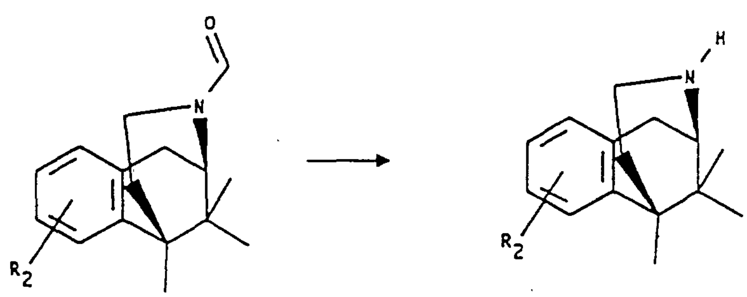

11 12

et

j) si on le souhaite, après libération de la base de benzomorphane libre, on convertit le substituant $R_2$, au cas où il représente un groupe alcoxy, par la voie d'un clivage d'éther, en une fonction hydroxyle libre et on isole le produit réactionnel, éventuellement sous forme de son sel d'addition d'acide de type 13, où la base correspond à la formule générale 1.

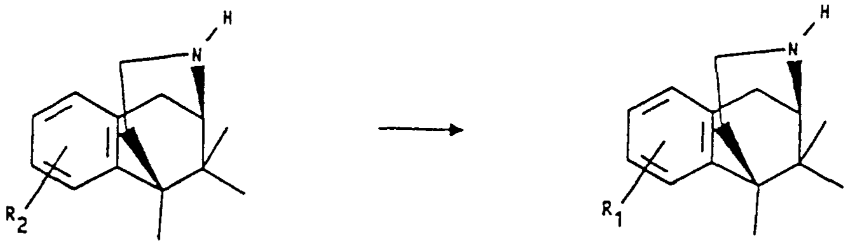

12 13

**2.** Procédé de préparation de norbenzomorphane de formule générale 1 selon la revendication 1

où

$R_1$ a la signification indiquée dans la revendication 1

caractérisé en ce que

a) on fait réagir un cyanure de benzyle de formule générale 2 dans laquelle $R_2$ a la signification indiquée dans la revendication 1 avec un ester d'acide bromoisobutyrique de formule générale 3 dans laquelle $R_3$ représente alkyle en $C_1$-$C_8$ ou benzyle, en présence d'un alkylhalogénosilane et de poudre de zinc dans un solvant inerte, on chauffe le mélange réactionnel, on le laisse refroidir après la fin de la réaction, on sépare la poudre de zinc et on ajoute au mélange réactionnel un réducteur sélectif à l'égard de la réduction des fonctions imino et on dilue le mélange réactionnel avec un alcanol puis on lui ajoute une solution aqueuse d'un composé à réaction basique, on sépare et concentre la phase organique, on reprend le résidu dans un solvant inerte, on extrait la solution obtenue avec la solution aqueuse d'un acide, on rend alcalins les extraits aqueux réunis avec un composé à réaction basique, on extrait cette solution alcaline avec un solvant organique immiscible à l'eau et on isole le dérivé d'ester d'acide 3-amino-2,2-diméthylbutanoïque résultant de formule générale 4

et

b) on soumet le dérivé d'ester d'acide 3-amino-2,2-diméthylbutanoïque de formule générale 4 avec un ester d'acide acrylique dans lequel le composant alcool $R_4$ a la signification d'un groupe alkyle en $C_1$-$C_8$ ou d'un groupe benzyle à une réaction d'addition de Michael dans un solvant inerte, on élimine le milieu réactionnel après la fin de la réaction et on isole le dérivé d'éthylester d'acide 3-(2-éthoxycarbonyléthyl)-amino-2,2-diméthylbutanoïque résultant de formule générale 5

et

c) on soumet le dérivé d'ester d'acide 3-(2-éthoxycarbonyléthyl)amino-2,2-diméthylbutanoïque de formule générale 5 ainsi préparé dans un solvant inerte en présence d'un composé à réaction basique aux conditions d'une condensation d'ester de Dieckmann, on élimine par distillation les constituants volatils du mélange réactionnel résultant de la réaction de cyclisation, puis on hydrolyse le mélange et on lui ajoute la solution aqueuse d'un composé à réaction acide, on ajoute au mélange résultant un solvant organique immiscible à l'eau et une solution aqueuse d'un composé à réaction basique, on concentre les extraits organiques réunis et on isole le dérivé de pipéridone résultant de formule générale 6

et

d) on saponifie et décarboxyle le dérivé de pipéridone 6 dans des conditions acides ou alcalines dans un solvant ou mélange de solvants polaire avec chauffage en le dérivé de 3,3-diméthylpipéridone correspondant de formule générale 7, on l'isole et, éventuellement, avec un acide on prépare le sel d'addition d'acide correspondant et on l'isole

et

e) on dissout le mélange de stéréoisomères ainsi obtenu, éventuellement après libération des bases libres énantiomères correspondantes, dans un milieu réactionnel inerte à l'égard de la séparation d'énantiomères, on ajoute un stéréoisomère approprié d'un acide organique approprié à la salification avec un stéréoisomère du mélange d'énantiomères, on isole le stéréoisomère voulu sous forme de son sel d'addition d'acide avec l'acide optiquement actif, on chauffe la liqueur mère contenant l'isomère non souhaité et on convertit alors thermiquement l'énantiomère non souhaité en le stéréoisomère voulu, on ajoute un acide organique optiquement actif, énantiomériquement pur, capable de former un sel d'addition d'acide et on ajoute le stéréoisomère souhaité présent ainsi sous forme de sel d'addition d'acide éventuellement avec addition d'un milieu qui se comporte comme un non-solvant à l'égard du sel souhaité, et on isole le sel et on répète éventuellement ce processus

et

f) après la libération à partir du sel d'addition d'acide énantiomériquement pur, on soumet à une réaction de Wittig le stéréoisomère pur ainsi obtenu dans un solvant inerte avec un réactif de Wittig générant un groupe méthylène en présence d'un composé à réaction basique dans un intervalle de température de 0 à 80°C, après la fin de la réaction on ajoute au mélange réactionnel de l'eau ainsi qu'un solvant organique immiscible à l'eau, on extrait la phase aqueuse totalement, on isole le produit réactionnel de type 9 ou, après addition d'un acide protonique, le stéréoisomère correspondant sous forme de son sel d'addition d'acide

et

g) éventuellement on libère d'abord à partir de son sel d'addition d'acide l'alcène 9 issu de la réaction de Wittig et on dissout la base libre de type 9 dans un solvant organique et on la soumet avec un agent de formylation à une réaction de formylation sur l'azote de pipéridine et on isole le produit réactionnel de type 10 ou son stéréoisomère correspondant

et

h) on dissout le composé formylé 10 ainsi obtenu, ou le stéréoisomère correspondant, dans un solvant inerte et on le fait réagir avec un acide de Lewis et on isole le produit de cyclisation de type 11 résultant de cette réaction

et

i) on dissout le dérivé de benzomorphane résultant de la réaction de cyclisation dans un solvant polaire et on le fait réagir avec un composé à réaction acide et on isole le norbenzomorphane déformylé de type 12 résultant de cette réaction sous forme de son sel d'addition d'acide après addition de l'acide minéral

et

j) si on le souhaite, après libération de la base de benzomorphane libre, on convertit le substituant $R_2$, au cas où il représente un groupe alcoxy, par la voie d'un clivage d'éther, en une fonction hydroxyle libre et on isole le produit réactionnel, éventuellement sous forme de son sel d'addition d'acide de type 13, où la base correspond à la formule générale 1.

**3.** Procédé de préparation de norbenzomorphane de formule générale 1 dans laquelle $R_1$ a la signification indiquée dans la revendication 1, selon la revendication 2,

caractérisé en ce que

a) on fait réagir un cyanure de benzyle de formule générale 2 dans laquelle $R_2$ a la signification indiquée dans la revendication 1 avec un ester d'acide bromoisobutylbutyrique de formule générale 3 dans laquelle $R_3$ représente alkyle en $C_1$-$C_6$, en présence d'un trialkylhalogénosilane et de poudre de zinc dans un éther et un halogénoalcane, on chauffe le mélange réactionnel, on le laisse refroidir après la fin de la réaction, on sépare la poudre de zinc et on ajoute au mélange réactionnel un dérivé complexe de borohydrure alcalin sélectif à l'égard de la réduction des fonctions imino et on dilue le mélange réactionnel avec un alcool en $C_1$-$C_4$ puis on lui ajoute une solution aqueuse d'ammoniac, on sépare et concentre la phase organique, on reprend le résidu dans un hydrocarbure aliphatique ou aromatique, on extrait la solution obtenue avec la solution aqueuse d'un acide minéral, on rend alcalins les extraits aqueux réunis avec une solution aqueuse d'ammoniac, on extrait cette solution alcaline avec un hydrocarbure halogéné et on isole le dérivé d'éthylester d'acide 3-amino-2,2-diméthyl-butanoïque résultant de formule générale 4

et

b) on soumet le dérivé d'ester d'acide 3-amino-2,2-diméthylbutanoïque de formule générale 4 avec un ester d'acide acrylique dans lequel le composant alcool $R_4$ a la signification d'un groupe alkyle en $C_1$-$C_8$ ou d'un groupe benzyle à une réaction d'addition de Michael dans un alcool en $C_1$-$C_4$ linéaire ou ramifié, on élimine le milieu réactionnel après la fin de la réaction et on isole le dérivé d'ester d'acide 3-(2-éthoxycarbonyléthyl) amino-2,2-diméthylbutanoïque résultant de formule générale 5

et

c) on soumet le dérivé d'ester d'acide 3-(2-éthoxycarbonyléthyl)amino-2,2-diméthylbutanoïque de formule générale 5 ainsi préparé dans un hydrocarbure aliphatique ou aromatique en présence d'un alcoolate alcalin d'un alcool en $C_1$-$C_4$ linéaire ou ramifié aux conditions d'une condensation d'ester de Dieckmann, on élimine par distillation les constituants volatils du mélange réactionnel résultant de la réaction de cyclisation, puis on hydrolyse le mélange et on lui ajoute la solution aqueuse d'un acide minéral, on ajoute au mélange résultant un dialkyléther immiscible à l'eau et une solution aqueuse d'ammoniac, on concentre les extraits organiques réunis et on isole le dérivé de pipéridone résultant de formule générale 6

et

d) on saponifie et décarboxyle le dérivé de pipéridone 6 dans un mélange d'un alcool en $C_1$-$C_4$ linéaire ou ramifié et d'eau en présence d'un hydroxyde alcalin ou d'un acide minéral avec chauffage en le dérivé de 3,3-diméthylpipéridone correspondant de formule générale 7, on isole le produit et, éventuellement, avec un

acide protonique on prépare le sel d'addition d'acide correspondant

et

e) on dissout le mélange de stéréoisomères ainsi obtenu, éventuellement après libération des bases libres énantiomères correspondantes, dans un alcool en $C_1$-$C_4$ linéaire ou ramifié, on ajoute l'énantiomère correspondant de l'acide malique, de l'acide tartrique, de l'acide phénylglycolique ou de l'acide camphosulfonique, on isole le stéréoisomère voulu sous forme de son sel d'addition avec l'acide optiquement actif, on chauffe la liqueur mère contenant l'isomère non souhaité et on convertit alors thermiquement l'énantiomère non souhaité en le stéréoisomère voulu, on ajoute un acide organique optiquement actif, énantiomériquement pur, capable de former un sel d'addition d'acide et on cristallise et isole le stéréoisomère souhaité présent ainsi sous forme de sel d'addition d'acide éventuellement par addition d'un alcool en $C_3$-$C_8$, et on répète éventuellement ce processus

et

f) après la libération à partir du sel d'addition d'acide énantiomériquement pur, on soumet à une réaction de Wittig le stéréoisomère pur ainsi obtenu dans un éther, éventuellement cyclique, avec un halogénure de méthyltriphénylphosphonium en présence d'un alcoolate alcalin dans un intervalle de température de 20 à 60°C, après la fin de la réaction on ajoute au mélange réactionnel de l'eau ainsi qu'un halogénoalcane, on extrait la phase aqueuse totalement, on isole le produit réactionnel de type 9 ou éventuellement le stéréoisomère correspondant après addition d'un acide protonique sous forme de son sel d'addition d'acide

et

g) éventuellement on libère d'abord à partir de son sel d'addition d'acide l'alcène 9 issu de la réaction de Wittig et on dissout la base libre de type 9 dans un composé alkylaromatique comme solvant et on la soumet avec un formiate d'alkyle à une réaction de formylation sur l'azote de pipéridine et on isole le produit réactionnel de type 10 ou son stéréoisomère correspondant

et

h) on dissout le composé formylé 10 ainsi obtenu, ou le stéréoisomère correspondant, dans un hydrocarbure halogéné et on le fait réagir avec un halogénure d'aluminium (III) et on isole le produit de cyclisation de type 11 résultant de cette réaction

et

i) on dissout le dérivé de benzomorphane 11 résultant de la réaction de cyclisation dans un alcanol et on le fait réagir avec la solution aqueuse d'un acide halogénhydrique et on isole le norbenzomorphane déformylé de type 12 résultant de cette réaction éventuellement sous forme de son sel d'addition d'acide après addition d'un acide protonique

et

j) si on le souhaite, après libération de la base de benzomorphane libre, on convertit le substituant $R_2$, au cas où il représente un groupe alcoxy, par la voie d'un clivage d'éther, en une fonction hydroxyle libre et on isole le produit réactionnel, éventuellement sous forme de son sel d'addition d'acide de type 13, où la base correspond à la formule générale 1.

**4.** Procédé de préparation de norbenzomorphane de formule générale 1 dans laquelle $R_1$ a la signification indiquée dans la revendication 1, selon la revendication 3,

caractérisé en ce que

a) on fait réagir un cyanure de benzyle de formule générale 2 dans laquelle $R_2$ a la signification indiquée dans la revendication 1 avec un ester d'acide bromoisobutylbutyrique de formule générale 3 dans laquelle $R_3$ représente alkyle en $C_1$-$C_6$, en présence de chlorotriméthylsilane et de poudre de zinc dans le dichlorométhane après dilution avec le tétrahydrofurane, on chauffe le mélange réactionnel, on le laisse refroidir après la fin de la réaction, on sépare la poudre de zinc et on ajoute au mélange réactionnel du cyanoborohydrure de sodium et on dilue le mélange réactionnel avec l'éthanol puis on lui ajoute une solution aqueuse d'ammoniac concentrée, on sépare et concentre la phase organique, on reprend le résidu dans le toluène, on extrait la solution obtenue avec de l'acide chlorhydrique 2N, on rend alcalins les extraits aqueux réunis avec une solution aqueuse d'ammoniac concentrée, on extrait cette solution alcaline avec du dichlorométhane et on isole le dérivé d'ester d'acide 3-amino-2,2-diméthylbutanoïque résultant de formule générale 4

et

b) on soumet le dérivé d'ester d'acide 3-amino-2,2-diméthylbutanoïque de formule générale 4 avec un ester d'acide acrylique dans lequel le composant alcool $R_4$ a la signification d'un groupe alkyle en $C_1$-$C_6$ à une réaction d'addition de Michael dans l'éthanol comme solvant, on élimine le milieu réactionnel après la fin de la réaction et on isole le dérivé d'ester d'acide 3-(2-éthoxycarbonyléthyl)amino-2,2-diméthylbutanoïque de formule générale 5

et

c) on soumet le dérivé d'ester d'acide 3-(2-éthoxycarbonyléthyl)amino-2,2-diméthylbutanoïque de formule générale 5 ainsi préparé dans le toluène en présence de tert.butanolate de potassium aux conditions d'une condensation d'ester de Dieckmann, on élimine par distillation les constituants volatils du mélange réactionnel résultant de la réaction de cyclisation, puis on hydrolyse le mélange et on lui ajoute de l'acide chlorhydrique concentré, on ajoute au mélange résultant du diéthyléther et une solution d'ammoniac concentrée, on concentre les extraits organiques réunis et on isole le dérivé de pipéridone résultant de formule générale 6

et

d) on saponifie et décarboxyle le dérivé de pipéridone 6 dans un mélange éthanol/eau en présence d'hydroxyde de sodium ou d'acide chlorhydrique ou d'acide sulfurique avec chauffage à la température de reflux en le dérivé de 3,3-diméthylpipéridone correspondant de formule générale 7, on isole le produit réactionnel et, éventuellement, avec l'acide chlorhydrique ou bromhydrique, on prépare l'halogénhydrate correspondant

et

e) on dissout dans l'éthanol le mélange de stéréoisomères ainsi obtenu, éventuellement après libération des bases libres énantiomères correspondantes, on ajoute l'énantiomère correspondant (forme D ou L) de l'acide tartrique, on isole le stéréoisomère voulu sous forme du tartrate correspondant, on chauffe la liqueur mère contenant l'isomère non souhaité et on convertit alors thermiquement l'énantiomère non souhaité en le stéréoisomère voulu, on ajoute de l'acide D- ou L-tartrique et on cristallise le stéréoisomère souhaité présent ainsi sous forme de tartrate correspondant, éventuellement par addition d'isopropanol, et on isole le précipité et on répète éventuellement ce processus

et

f) après la libération à partir du sel d'addition d'acide énantiomériquement pur, on soumet à une réaction de Wittig le stéréoisomère pur ainsi obtenu dans le tétrahydrofurane avec le bromure de méthyltriphénylphosphonium en présence de tert.butanolate de potassium à une température de 40°C, après la fin de la réaction on ajoute au mélange réactionnel de l'eau ainsi que du dichlorométhane, on extrait la phase aqueuse totalement, on isole le produit réactionnel de type 9 ou le stéréoisomère correspondant sous forme de son halogénhydrate

et

g) éventuellement on libère d'abord à partir de son sel d'addition d'acide l'alcène 9 issu de la réaction de Wittig et on dissout la base libre de type 9 dans le toluène et on la soumet avec le formiate de n-butyle à une réaction de formylation sur l'azote de pipéridine et on isole le produit réactionnel de type 10 ou son stéréoisomère correspondant

et

h) on dissout le composé formylé 10 ainsi obtenu, ou le stéréoisomère correspondant, dans le dichlorométhane et on le fait réagir à une température ne dépassant pas -5°C avec le chlorure d'aluminium (III) et on isole le produit de cyclisation de type 11 résultant de cette réaction

et

i) on dissout le dérivé de benzomorphane 11 résultant de la réaction de cyclisation dans le n-propanol et on le fait réagir avec de l'acide chlorhydrique concentré et on isole le norbenzomorphane déformylé de type 12 résultant de cette réaction sous forme de son chlorhydrate

et

j) si on le souhaite, après libération de la base de benzomorphane libre, on convertit le substituant $R_2$, au cas où il représente un groupe alcoxy, par la voie d'un clivage d'éther, en une fonction hydroxyle libre et on isole le produit réactionnel, éventuellement après addition d'acide chlorhydrique ou bromhydrique, sous forme de son halogénhydrate de formule générale 13, où la base correspond à la formule générale 1.

**5.** Procédé de préparation de norbenzomorphane de formule générale 1 dans laquelle $R_1$ représente un groupe hydroxyle qui se trouve en position 3', selon la revendication 4, caractérisé en ce que

a) on fait réagir un cyanure de benzyle de formule générale 2 dans laquelle $R_2$ a la signification d'un groupe méthoxy qui se trouve en position 3, avec l'éthylester d'acide bromoisobutylbutyrique (3) ($R_3 = C_2H_5$) en présence de chlorotriméthylsilane et de poudre de zinc dans le dichlorométhane après dilution avec le tétrahydrofurane, on chauffe le mélange réactionnel, on le laisse refroidir après la fin de la réaction, on sépare la poudre de zinc et on ajoute au mélange réactionnel du cyanoborohydrure de sodium et on dilue le mélange réactionnel avec l'éthanol puis on lui ajoute une solution aqueuse d'ammoniac concentrée, on sépare et concentre la phase organique, on reprend le résidu dans le toluène, on extrait la solution obtenue avec de l'acide chlorhydrique 2N, on rend alcalins les extraits aqueux réunis avec une solution aqueuse d'ammoniac concentrée, on extrait cette solution alcaline avec du dichlorométhane et on isole l'éthylester d'acide 3-amino-4-(3-méthoxyphényl)-2,2-diméthylbutanoïque (4, $R_2$ = 3-$OCH_3$) résultant

et

b) on soumet l'éthylester d'acide 3-amino-4-(3-méthoxyphényl)-2,2-diméthylbutanoïque (4, $R_2$ = 3-$OCH_3$) avec l'acrylate d'éthyle ($R_4$ = $C_2H_5$) à une réaction d'addition de Michael dans l'éthanol comme solvant, on élimine le milieu réactionnel après la fin de la réaction et on isole l'éthylester d'acide 3-(2-éthoxycarbonyléthyl)amino-4-(3-méthoxyphényl)-2-diméthylbutanoïque (5, $R_2$ = 3-$OCH_3$)

et

c) on soumet l'éthylester d'acide 3-(2-éthoxycarbonyléthyl)amino-4-(3-méthoxyphényl)-2-diméthylbutanoïque (5, $R_2$ = 3-$OCH_3$) dans le toluène en présence de tert.butanolate de potassium aux conditions d'une condensation d'ester de Dieckmann, on élimine par distillation les constituants volatils du mélange réactionnel résultant de la réaction de cyclisation, puis on hydrolyse le mélange et on lui ajoute de l'acide chlorhydrique concentré, on ajoute au mélange résultant du diéthyléther et une solution d'ammoniac concentrée, on concentre les extraits organiques réunis et on isole la 5-carboéthoxy-3,3-diméthyl-2-(3-méthoxyphényl)méthyl-4-pipéridone (6, $R_2$ = 3-$OCH_3$)

et

d) on saponifie et décarboxyle la 5-carboéthoxy-3,3-diméthyl-2-(3-méthoxyphényl)méthyl-4-pipéridone (6, $R_2$ = 3-$OCH_3$) dans un mélange éthanol/eau en présence d'hydroxyde de sodium ou d'acide chlorhydrique ou d'acide sulfurique avec chauffage à la température de reflux en le chlorhydrate de 2-(3-méthoxyphényl)méthyl-3,3-diméthyl-4-pipéridone (7, $R_2$ = m-$CH_3O$), on isole le produit réactionnel et on précipite le chlorhydrate de 2-(3-méthoxyphényl)méthyl-3,3-diméthyl-4-pipéridone correspondant

et

e) on dissout dans l'éthanol le mélange de stéréoisomères ainsi obtenu du chlorhydrate de 2-(3-méthoxyphényl)méthyl-3,3-diméthyl-4-pipéridone de type 7 ($R_2$ = 3-$OCH_3$) après libération des bases libres énantiomères correspondantes, on ajoute de l'acide D-(-)-tartrique, on isole l'hydrogénotartrate de (+)-2-(3-méthoxyphényl) méthyl-3,3-diméthyl-4-pipéridonium de type 8A ($R_2$ = 3-$OCH_3$) souhaité, on chauffe la liqueur mère contenant l'isomère non souhaité et on convertit alors thermiquement la (-)-2-(3-méthoxyphényl)méthyl-3,3-diméthyl-4-pipéridone (8B, $R_2$ = 3-$OCH_3$) non souhaitée en la (+)-2-(3-méthoxyphényl)méthyl-3,3-diméthyl-4-pipéridone (8A) souhaitée, on ajoute de l'acide D-(-)-tartrique et on cristallise l'hydrogénotartrate de (+)-2-(3-méthoxyphényl)méthyl-3,3-diméthyl-4-pipéridonium de type 8A ($R_2$ = 3-$OCH_3$) souhaité par addition d'isopropanol, et on isole le précipité et on répète ce processus

et

f) après la libération à partir du sel d'addition d'acide énantiomériquement pur, on soumet à une réaction de Wittig l'hydrogénotartrate de (+)-2-(3-méthoxyphényl)méthyl-3,3-diméthyl-4-pipéridonium de type 8A ($R_2$ = 3-$OCH_3$) pur ainsi obtenu dans le tétrahydrofurane avec le bromure de méthyltriphénylphosphonium en présence de tert.butanolate de potassium à une température de 40°C, après la fin de la réaction on ajoute au mélange réactionnel de l'eau ainsi que du dichlorométhane, on extrait la phase aqueuse totalement, on isole la (+)-2-(3-méthoxyphényl)méthyl-3,3-diméthyl-4-méthylène-pipéridine (9) et on la convertit avec l'acide chlorhydrique en le chlorhydrate de (+)-2-(3-méthoxyphényl)méthyl-3,3-diméthyl-4-méthylène-pipéridine de type 9 ($R_2$ = 3-$OCH_3$)

et

g) on libère à partir de son chlorhydrate la (3-méthoxyphényl)méthyl-3,3-diméthyl-4-méthylène-pipéridine (9) ($R_2$ = 3-$OCH_3$) issue de la réaction de Wittig et on dissout la base libre dans le toluène et on la soumet avec le formiate de n-butyle à une réaction de formylation sur l'azote de pipéridine et on isole la (+)-N-formyl-2-(3-méthoxyphényl)méthyl-3,3-diméthyl-4-méthylène-pipéridine de type 10 ($R_2$ = 3-$OCH_3$)

et

h) on dissout la (+)-N-formyl-2-(3-méthoxyphényl)méthyl-3,3-diméthyl-4-méthylène-pipéridine de type 10 ($R_2$ = 3-$OCH_3$) dans le dichlorométhane et on la fait réagir avec le chlorure d'aluminium (III) et on isole le (-)-2-formyl-3'-méthoxy-5,9,9-triméthyl-6,7-benzomorphane de type 11 ($R_2$ = 3'-$CH_3O$) résultant de cette réaction

et

i) on dissout le (-)-2-formyl-3'-méthoxy-5,9,9-triméthyl-6,7-benzomorphane de type 11 ($R_2$ = 3'-$CH_3O$) résultant de la réaction de cyclisation dans le n-propanol et on le fait réagir avec de l'acide chlorhydrique concentré et on convertit le(-)-3'-méthoxy-5,9,9-triméthyl-6,7-benzomorphane de type 12 ($R_2$ = 3'-$CH_3O$) résultant de cette réaction en le chlorhydrate avec l'acide chlorhydrique

et

j ) après libération du (-)-3'-méthoxy-5,9,9-triméthyl-6,7-benzomorphane de type 12 ($R_2$ = 3'-$CH_3O$) libre à partir du chlorhydrate, on convertit la fonction 3'-méthoxy avec l'acide bromhydrique aqueux dans des conditions de reflux en une fonction hydroxyle libre et on convertit le (-)-3'-hydroxy-5,9,9-triméthyl-6,7-benzomorphane de type 1 avec l'acide bromhydrique en le bromhydrate de (-)-3'-hydroxy-5,9,9-triméthyl-6,7-benzomorphane correspondant de formule générale 13, ($R_2$ = 3'-OH), qui correspond à la formule générale 1.

**6.** Procédé selon l'une des revendications 1 à 5 caractérisé en ce que, dans le cas où $R_1$ a la signification de nitro, cyano, -$NH_2$, -NH(alkyle en $C_1$-$C_8$, -N(alkyle en $C_1$-$C_8$)$_2$, où les restes alkyle peuvent être identiques ou différents, -NH-acyl-(alkyle en $C_1$-$C_8$) où acyle représente benzoyle ou un reste alkylcarbonyle avec un reste alkyle inférieur linéaire ou ramifié de 1 à 6 atomes de carbone, où le reste alkyle peut éventuellement être substitué par un ou plusieurs atomes d'halogène qui peuvent être identiques ou différents,

un composé de formule générale 12 dans laquelle $R_2$ représente l'hydrogène est converti d'une manière connue en soi en le composé fonctionnalisé de manière correspondante de formule générale 13.